# EUROPÄISCHE PATENTSCHRIFT

(11) **EP 1 220 921 B1**
(45) Veröffentlichungstag und Bekanntmachung des Hinweises auf die Patenterteilung: **23.04.2008**
(21) Anmeldenummer: 00982993.8
(22) Anmeldetag: 11.10.2000
(51) Int. Cl.: C12N 15/12, C12N 15/11, C07K 14/47, C07K 16/18, C12Q 1/68, G01N 33/53

(54) **NUKLEINSÄURESEQUENZEN VON HYPERPLASIEN UND TUMOREN DER SCHILDDRÜSE**
NUCLEIC ACID SEQUENCES OF HYPERPLASIA AND TUMOURS OF THE THYROID
SEQUENCES D'ACIDES NUCLEIQUES D'HYPERPLASIES ET DE TUMEURS DE LA GLANDE THYROIDE

(30) Priorität: 12.10.1999 DE 19949179
(43) Veröffentlichungstag der Anmeldung: 10.07.2002
(73) Patentinhaber: Universität Bremen, 28359 Bremen (DE)
(72) Erfinder: BULLERDIEK, Jörn, 28350 Bremen (DE); RIPPE, Volkhard, 27321 Emtinghausen (DE); MEIBOOM, Maren, 28203 Bremen (DE); BELGE, Gazanfer, 28329 Bremen (DE)
(74) Vertreter: Bohmann, Armin K.
(86) Internationale Anmeldenummer: PCT/DE2000/003600
(87) Internationale Veröffentlichungsnummer: WO 2001/027265

(56) Entgegenhaltungen:
- DATABASE EMBL [Online] 8. Oktober 1999 (1999-10-08) " Homo sapiens chromosome 19 clone CTB-167G5, WORKING DRAFT SEQUENCE, 9" retrieved from EBI Database accession no. ac011487 XP002161621
- GOLDWURM S ET AL: "Identification of a novel Krueppel-related zinc finger gene (ZNF184) mapping to 6p21.3." GENOMICS, Bd. 40, Nr. 3, 1997, Seiten 486-489, XP000982502 ISSN: 0888-7543
- N TOMMERUP ET AL: "Isolation and fine mapping of 16 novel human zinc finger-encoding cDNAs identify putative candidate genes for developmental and malignant disorders" GENOMICS,US,ACADEMIC PRESS, SAN DIEGO, Bd. 27, Nr. 2, 20. Mai 1995 (1995-05-20), Seiten 259-264, XP002117526 ISSN: 0888-7543
- DATABASE SWALL [Online] 1. November 1998 (1998-11-01) SHANNON M. ET AL.: "ZINC FINGER PROTEIN 54." retrieved from EBI Database accession no. O88631 XP002161622
- DATABASE SWALL [Online] 1. Mai 1999 (1999-05-01) AGATA Y. ET AL.: " KRAB-CONTAINING ZINC-FINGER PROTEIN KRAZ1." retrieved from EBI Database accession no. Q9Z117 XP002161623
- RIPPE VOLKHARD ET AL: "A KRAB zinc finger protein gene is the potential target of 19q13 translocation in benign thyroid tumors." GENES CHROMOSOMES & CANCER, Bd. 26, Nr. 3, November 1999 (1999-11), Seiten 229-236, XP000982498 ISSN: 1045-2257

## Beschreibung

Die vorliegende Erfindung betrifft eine Nukleinsäure, die für ein Polypeptid codiert, wobei das Polypeptid eine KRAB-Domäne und mindestens ein Zinkfinger-Motiv umfaßt. Weiterhin betrifft die Erfindung Vektoren, Polypeptide, Zellen, Antikörper, Ribozyme, deren Verwendungen zur Diagnose und/oder Therapie von Funktionsstörungen der Schilddrüse und/oder Hyperplasien der Schilddrüse und/oder Schilddrüsentumoren sowie ihre Verwendungen zur Herstellung eines Medikaments, pharmazeutische Zusammensetzungen; Kits sowie Verfahren zum Nachweis von Funktionsstörungen der Schilddrüse, Hyperplasien der Schilddrüse und/oder Tumoren der Schilddrüse, Primer zur Darstellung der Nukleinsäure, Verfahren zur Darstellung der Nukleinsäure sowie eine pharmazeutische Zusammensetzung.

Die Bedeutung der Schilddrüse infolge ihrer Hormonproduktion für die Kontrolle des Wachstums und der Entwicklung des Körpers ist in der Medizin ebenso wie die mit einer Funktionsstörung dieser Drüse zumindest in einigen Fällen im wahrsten Sinne des Wortes augenfälligen Veränderungen seit langem bekannt.

Hinsichtlich der Pathogenese von Strumen und Schilddrüsentumoren allgemein ist es insbesondere auf molekulare Ebene noch nicht gelungen, eine umfassende Vorstellung zu entwickeln. Derzeit werden zwei Konzepte diskutiert, wobei eines davon ausgeht, daß das hyperplastische Gewebe als infolge chronischer Stimulation durch ein trophisches Hormon, was letztendlich das Wachstum polyklonaler Knoten zur Folge hat, bedingt betrachtet wird. Dieses Konzept wird als nicht-neoplastische endokrine Hyperplasie (NNEH) bezeichnet. Das zweite Konzept geht davon aus, daß die Knoten echte klonale Tumoren darstellen.

Im Falle der Schilddrüse hat sich gezeigt, daß das auf andere Drüsen anwendbare einfache Konzept der nicht-neoplastischen endokrinen Hyperplasie nicht anzuwenden ist Eine Über sicht über die derzeit auf diesem Gebiet angestellten Überlegungen findet sich bei Studer H (1995); Endocrine Reviews, Vol. 16, No. 4, Seiten 411-426.

Für die Behandlung von Strumen und Schilddrüsentumoren ist somit eine frühzeitige Diagnose erforderlich, um geeignete therapeutische Konzepte zur Anwendung gelangen zu lassen.

Der Eintrag in der Datenbank mit der Zugangsnummer AC011487 offenbart die Sequenz des BAC-Klons CTB-16765. Der Eintrag bestand zum Veröffentlichungszeitraum aus etwa 70 nicht geordneten Sequenzfragmenten (contigs) und enthielt im Übrigen keinerlei Angaben über die in der angegebenen Sequenz enthaltenen Gene oder Genfragmente.

Die Veröffentlichung von Tommerugs & Vissing (1995), Genomics 27: 259-264 offenbart Zinkfinger-Protein-codierende DNA-Sequenzen.

Der vorliegenden Erfindung liegt die Aufgabe zugrunde, Mittel zur Diagnose und Therapie von Funktionsstörungen der Schilddrüse, Hyperplasien der Schilddrüse und Tumoren der Schilddrüse auf der molekularen Ebene bereitzustellen, insbesondere sollten Nukleinsäuresequenzen bereitgestellt werden, die an den Pathogenitätsmechanimnen beteiligt und geeignet sind, diese weitergehend zu untersuchen sowie solche, die auf die Pathogenitätsmechanismen einwirken oder diese beeinflussen können.

Darüber hinaus sollen darauf aufbauende Medikamente sowie allgemein pharmazeutische Zusammensetzungen bereitgestellt werden.

Gleiches gilt für die aus den Nukleinsäuresequenzen abgeleiteten Polypeptide sowie darauf gerichtete Antikörper, Ribozyme und diese enthaltende pharmazeutische Zusammensetzungen.

Desweiteren sollen Kits zur Diagnose und/oder Therapie von Funktionsstörungen der Schilddrüse, Hyperplasien der Schilddrüse und Tumoren der Schilddrüse ebenso wie Verfahren zum Nachweisen dieser zur Verfügung gestellt werden.

Erfindungsgemäß wird die Aufgabe durch den Gegenstand der unabhängigen Ansprüche gelöst. Bevorzugte Ausführungsformen ergeben sich aus den Unteransprüchen.

Im folgenden soll hierin unter Tumoren sowohl maligne als auch benigne Tumoren verstanden werden.

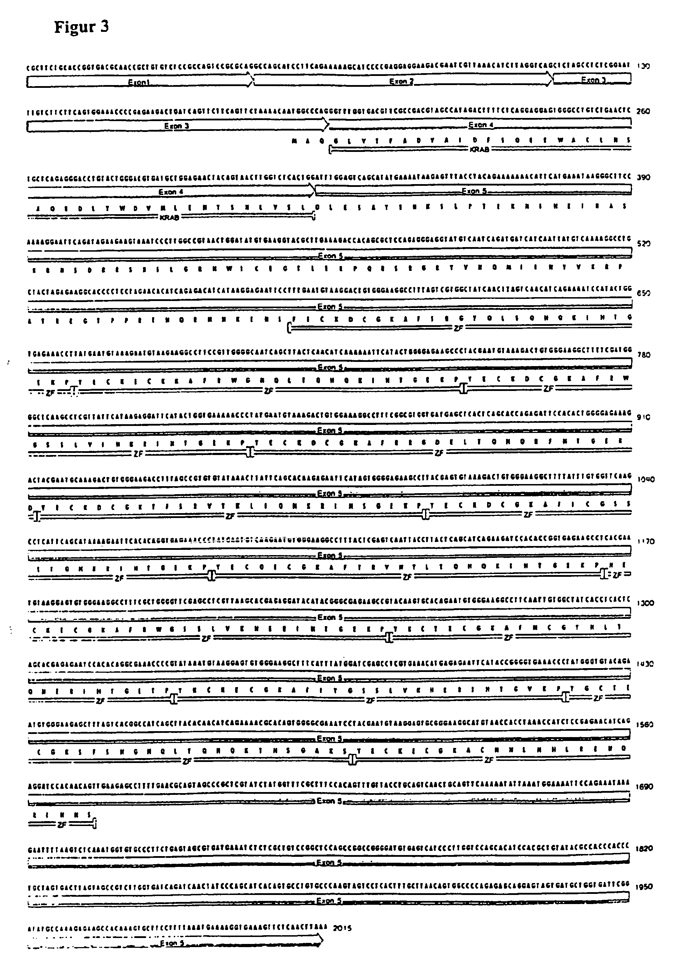

Der Erfindung liegt die überraschende Erkenntnis zugrunde, daß die in Gewebeproben von follikulären Schilddrüsentumoren, einschließlich Schilddrüsenkarzinomen, und Strumen beobachteten Chromosomentranslokationen auf dem menschlichen Chromosom 19 die chromosomale Bande 19q13 einbeziehen, und daß insbesondere alle Bruchpunkte sich einer bestimmten Nukleinsäuresequenz innerhalb der Bande 19q13 zuordnen lassen, wie hierin offenbart wird. Insbesondere scheinen die hierin offenbarten erfindungsgemäßen Nukleinsäuren Target-Nukleinsäuren für die Aberrationen 19q13, insbesondere bei epithelialen Tumoren, zu sein. Target-Nukleinsäure bedeutet dabei, daß die beobachteten chromosomalen Aberrationen in der erfindungsgemäßen Nukleinsäure erfolgen oder in deren unmittelbaren Nachbarschaft. In diesem Zusammenhang bedeutet unmittelbare Nachbarschaft in einer Entfernung von etwa 200 kbp. Als Bezugspunkt kann dabei sowohl das 3'-Ende der erfindungsgemäßen Nukleinsäure als auch das 3'-Ende der in Fig. 3 dargestellten cDNA (SEQ ID NO: 3) sein.

Weiterhin wurde überraschend gefunden, daß es möglich ist, Zellen zu erzeugen, die die Chromosomenveränderung, d.h. die Chromosomentranslokationen in der Bande 19q13, aufweisen, wie in Beispiel 2 gezeigt wird.

Schließlich wurde überraschenderweise noch festgestellt, daß die Sequenz, innerhalb der die Bruchpunkte der Chromosomentranslokationen liegen, im Schilddrüsengewebe und insbesondere im Schilddrüsentumorgewebe exprimiert wird.

Im Zusammenhang mit der vorliegenden Erfindung wird unter Zinkfinger-Domäne mindestens ein Zinkfinger-Motiv und insbesondere eine Ansammlung von mindestens zwei Zinkfinger-Motiven verstanden, obgleich ein jedes Zinkfinger-Motiv bereits für sich genommen dem Kriterium einer Domäne genügt, d.h. die Aminosäuresequenz des Zinkfinger-Motivs kann sich bereits als solches in eine stabil Tertiärstruktur falten. Insbesondere gilt es festzustellen, daß im Zusammenhang mit der vorliegenden Erfindung die als Zinkfinger-Domäne bezeichnete Struktur 12 Zinkfinger-Motive umfaßt. Die genaue Lokalisation dieser Zinkfinger-Motive ergibt sich auch aus Fig. 3 (dort werden die einzelnen Zinkfinger-Motive mit "ZF" bezeichnet). Des weiteren finden sich in Beispiel 12 die genauen Nucleotidgrenzen für die einzelnen Zinkfinger-Motive.

Genauer gesagt kann eine derartige Nukleinsäuresequenz eine oder mehrere der Nukleinsäuresequenzen umfassen, wie sie für eine Aminosäuresequenz kodieren, die durch eine Nukleinsäuresequenz der Nukleotidpositionen 577 - 660, 661 - 744, 745 - 828, 829 - 912, 913 - 996, 997 - 1080, 1081 - 1164, 1165 - 1248, 1249 - 1332, 1333 - 1416, 1417 - 1500 oder 1501 - 1575 (wie auch dargestellt in Beispiel 12) von Fig. 3 kodiert wird. Mit anderen Worten, die erfindungsgemäße Nukleinsäure kann die Nukleinsäure von Position 577 - 660 umfassen oder weitere der durch die vorstehenden Nukleotidpositionen definierten Sequenzen. Weiterhin sind auch jene Sequenzen umfaßt, die die durch die oben genannten Nukleinsäurepositionen definierten Aminosäuresequenzen kodieren und von der in Fig. 3 angegebenen Nukleinsäuresequenz abweichen in Folge der Degeneriertheit des genetischen Codes. Schließlich sollen erfindungsgemäß auch jene Nukleinsäuresequenzen umfaßt sein, die, bspw., die für die KRAB-Sequenz nach SEQ ID NO: 1 codierende Nukleinsäure und die Nukleinsäure entsprechend den Sequenzpositionen 577 bis 660 (das erste Zinkfinger-Motiv der in Fig. 3 dargestellten cDNA und damit des Gens RITA) umfaßt. Bezeichnet man die für die KRAB-Domäne nach SEQ ID NO: 1 codierende Nukleinsäure mit A und die die in Fig. 3 dargestellten 12 Zinkfinger-Motive kodierenden Nukleinsäuren mit B1, B2, etc. bis B 12, so umfaßt die erfindungsgemäße Nukleinsäure insbesondere jegliche Kombination von A mit mindestens einem Element der aus B1 bis B12 gebildeten Gruppe. Dabei kann auch vorgesehen sein, daß die aus den Elementen B1 bis B12 gebildete Gruppe auch solche Elemente umfaßt, die wiederum Kombinationen mindestens zweier der Elemente B1 bis B 12 sind.

Gemäß der vorliegenden Erfindung kann die erfindungsgemäße Nukleinsäure, die für ein das zelluläre Geschehen in Schilddrüsengewebe beeinflussendes Polypeptid codiert, wobei das Polypeptid eine KRAB-Domäne und mindestens ein Zinkfinger-Motiv umfaßt, und die KRAB-Domäne die Aminosäuresequenz von SEQ ID NO:1 umfaßt und das Zinkfinger-Motiv ein solches ist, wie es in der Zinkfinger-Domäne mit der Aminosäuresequenz von SEQ ID NO:2 umfaßt ist, in verschiedenen Ausführungsformen verschieden ausgebildet sein.

Mit den vorliegenden Nukleinsäuresequenzen eröffnen sich somit neue Möglichkeiten sowohl der Diagnose als auch der Therapie sowie der Untersuchung der mit dem Auftreten von Strumen und Schilddrüsentumoren bzw. Schilddrüsenkarzinomen verbundenen Mechanismen. Insbesondere wird durch Kenntnis der Sequenz die Möglichkeit gegeben, auf molekularer Ebene geeignete diagnostische bzw. therapeutische Mittel i.S. von - pharmazeutischen - Zusammensetzungen sowie Medikamenten mittelbar oder unmittelbar einzusetzen.

Die erfindungsgemäßen Nukleinsäuresequenzen können in für den Fachmann auf diesem Gebiet bekannter Art und Weise für die Gestaltung geeigneter diagnostisch und therapeutisch einsetzbarer Mittel im obigen Sinne sowie Kits und Verfahren verwendet werden.

Unter Nukleinsäure sollen dabei sowohl DNA-Sequenzen als auch RNA-Sequenzen, einschließlich Hybriden davon, verstanden werden, einschließlich hiervon abgeleiteter Derivate mit geändertem Rückgrat. Dies schließt auch ein, daß die Nukleinsäuren einzelsträngig, doppelsträngig oder als Tripelstruktur vorliegen.

Es kann dabei ebenfalls vorgesehen sein, daß sich die Strängigkeit der DNA, d.h. ob diese beispielsweise einzelsträngig oder doppelsträngig vorliegt, über die Länge der Nukleinsäuresequenz ändert.

Insbesondere kann auch vorgesehen sein, daß die erfindungsgemäße Nukleinsäuresequenz nicht vollständig, sondern als Fragment vorliegt.

Desweiteren ist es innerhalb des Umfangs der hierin vorliegenden Offenbarung, daß die Nukleinsäuresequenzen in mutierter Form vorliegen können. Unter Mutation sollen hierin alle dem Fachmann bekannten Mutationen, die innerhalb einer Nukleinsäuresequenz auftreten können, verstanden werden, einschließlich Punktmutationen sowie Nichtpunktmutationen, d.h. Inversionen, Insertionen und Deletionen.

Insbesondere unter dem Aspekt der Interaktion der erfindungsgemäßen Nukleinsäure mit anderen Nukleinsäuren soll hierin das Kriterium der Hybridisierung herangezogen werden, welches in der Technik allgemein anerkannt ist. Es wird anerkannt werden, daß durch Wahl geeigneter Hybridisierungsbedingungen die Stringenz der Hybridisierung innerhalb eines bestimmten Bereiches verändert werden kann und somit eine Hybridisierung der erfindungsgemäßen Nukleinsäuresequenzen an Nukleinsäuresequenzen möglich wird, deren Grad der Abweichung von der vollständig korrespondierenden, d.h. komplementären Sequenz schwanken kann.

Unter Nukleinsäuresequenz im erfindungsgemäßen Sinne soll auch jene Sequenz verstanden werden, die mit einer der erfindungsgemäßen Sequenzen hybridisieren würde, wenn nicht die Degeneration des genetischen Codes wäre. Dies bedeutet, daß mit Blick auf den in der erfindungsgemäßen Nukleinsäuresequenz vorhandenen offenen Leserahmen, bzw. die offenen Leserahmen, diese(r) unter Verwendung des genetischen Codes in eine Aminosäuresequenz translatiert werden kann/können. Infolge der Degeneration des genetischen Codes ist es jedoch möglich, ausgehend von der solchermaßen erhaltenen Aminosäuresequenz, wieder unter Verwendung des genetischen Codes eine Nukleinsäuresequenz zu gewinnen, die so verschieden ist, daß sie für sich genommen, mit der für die Bestimmung der Aminosäuresequenz herangezogenen Nukleinsäuresequenz möglicherweise nicht mehr hybridisieren kann.

Ausgehend von den hierin offenbarten Nukleinsäuren ist es für den Fachmann möglich, eine geeignete antisense-Nukleinsäure, inbesondere antisense-RNA zu erzeugen, die mit den erfindungsgemäßen Nukleinsäuresequenzen interagieren kann. Aufgrund dieser Interaktion ist eine direkte Beeinflussung der die Nukleinsäuresequenzen involvierenden Vorgänge möglich. Diese Interaktion kann beispielsweise auf der Ebene der Transkription ebenso wie auf der Ebene der Translation erfolgen.

Unter Nukleinsäuresequenzen sollen hierin allgemein Nukleinsäuresequenzen verstanden werden, die durch Isolierung aus dem genannten Gewebe in situ oder ex vivo, beispielsweise aus entsprechenden Zell-, Gewebe- oder Organkulturen, gewonnen werden können. Es sollen hierin jedoch auch entsprechende Nukleinsäuresequenzen verstanden werden, die aus Genbanken, insbesondere menschlichen Genbanken und noch bevorzugter Genbanken des menschlichen Chromosoms 19, isolierbar sind. Desweiteren soll hierin der Begriff Nukleinsäuresequenzen auch solche umfassen, die mittels geeigneter Synthesetechniken herstellbar sind, einschließlich der Polymerasekettenreaktion, und anderen im Stand der Technik bekannten, biochemischen und chemischen Syntheseverfahren.

Die erfindungsgemäßen Sequenzen können darüber hinaus modifiziert vorliegen.

Unter Modifikation soll hierin unter anderem Fragmentierung, Insertion, Deletion und Reversion von (Teil-)Sequenzen der erfinderischen Nukleinsäuresequenzen verstanden werden. Dies schließt auch die Insertion anderer Nukleinsäuresequenzen ein. Diese Nukleinsäuresequenzen können beispielsweise für bestimmte Domänen codieren, als Spacer dienen sowie Elemente zur Translations- und Transkriptionsregulierung dienen.

Weiterhin können die erfindungsgemäßen Nukleinsäuren dergestalt modifiziert sein, daß sie Sequenzen oder Moleküle umfassen, die eine Interaktion mit anderen Molekülen erlauben. Dies kann beispielsweise in Form einer Bindungsstelle an einen festen Träger oder einer Sequenz, die die Bindung an ein Nukleinsäure-bindendes Protein vermittelt, erfolgen.

Weiterhin können die erfindungsgemäßen Nukleinsäuresequenzen markiert sein. Hierin soll unter Markierung grundsätzlich sowohl direkte als auch indirekte Markierung verstanden werden. Eine Markierung kann unter Verwendung der im Stand der Technik bekannten Markierungen sowie Markierungsverfahren erfolgen und schließt radioaktive, nicht-radioaktive und Fluoreszenzmarkierung ein. Nicht-radioaktive Markierungen umfassen, unter anderem, die Verwendung von Digoxygenin, Avidin, Streptavidin und Biotin.

Unter Vektor sollen hierin insbesondere rekombinante Vektoren, wie sie in der Technik bekannt sind, verstanden werden. Derartige Vektoren schließen, unter anderem, virale Vektoren, wie beispielsweise adenovirale oder retrovirale Vektoren und Phagensysteme, sowie Plasmidvektoren, einschließlich Cosmidvektoren, und künstliche Chromosomen, die in prokaryontischen und/oder eukaryontischen Systemen verwendet werden können, ein.

Neben den erfindungsgemäßen Nukleinsäuresequenzen können die erfindungsgemäßen Vektoren weitergehende Elemente umfassen, die im Stand der Technik bekannt sind. Die jeweiligen Elemente, wie, beispielsweise, Promotoren, Terminatoren und Enhancer, werden entsprechend dem jeweiligen Wirtszellsystem in für den Fachmann bekannter Art und Weise ausgewählt. Insbesondere ist hierbei an die Auswahl eines geeigneten eukaryontischen Promotors und eines induzierbaren Promotors gedacht. Neben den genannten Elementen ist es auch noch möglich, daß in derartigen Vektoren solche Elemente enthalten sind, die dazu führen, daß zumindest die erfindungsgemäße Nukleinsäuresequenz, oder ein Teil davon, in das Genom des Wirtszellsystems integriert wird.

Es ist auch möglich, daß mindestens eines der genannten Elemente im Leserahmen ("in-frame") mit mindestens einem offenen Leserahmen der erfindungsgemäßen Nukleinsäuresequenzen verbunden ist, und ganz besonders vorteilhaft ist es, wenn mittels eines zusätzlich eingeführten Promotors die Transkriptionsrate des speziellen offenen Leserahmens kontrolliert wird, wobei der Promotor dann typischerweise in geeignetem Abstand und "in-frame" mit dem offenen Leserahmen ist.

Dabei kann weiterhin vorgesehen sein, daß ein offener Leserahmen der erfindungsgemäßen Nukleinsäuresequenzen, bevorzugt unter den vorgenannten Bedingungen, eine Signalsequenz aufweist, die eine Translokation des vom offenen Leserahmen codierten Genproduktes über eine Membran, und ggf. infolgedessen eine weitergehende Modifikation des Genprodukts erlaubt. Derlei Signalsequenzen schließen solche für den Transport des synthetisierten Proteins zum Endoplasmatischen Retikulum, Golgi-Apparat, zu Lysosomen, zu Zellorganellen, wie Mitochondrien und Chloroplasten, sowie zum Zellkern ein. Das solchermaßen mögliche Durchlaufen verschiedener zellulärer Kompartimente erlaubt eine posttranslationale Modifikation und damit ggf. eine weitergehende vorteilhafte Ausbildung des Genproduktes.

Neben Signalsequenzen kann ein derartiges Konstrukt auch zusätzliche Nukleinsäuresequenzen enthalten, die dazu führen, daß das Genprodukt eines offenen Leserahmens der erfindungsgemäßen Nukleinsäuresequenzen ein Fusionsprotein ausbildet, wobei der anfusionierte Teil einer Domäne eines anderen Proteins entsprechen kann und z.B. der Detektion des Genproduktes des offenen Leserahmens der erfindungsgemäßen Sequenzen dient, oder der Interaktion mit anderen Molekülen bzw. Strukturen im biologischen System, wobei das biologische System bevorzugt die Zelle ist.

Neben den vorgenannten und den weiteren sich für den Fachmann aus der Beschreibung ergebenden Vorteile der erfindungsgemäßen Nukleinsäuresequenzen sind diese auch den aus den genannten Nukleinsäuresequenzen ableitbaren Polypeptiden inhärent. Diese Polypeptide können zum einen direkt aus einem offenen Leserahmen der erfindungsgemäßen Nukleinsäuresequenz abgeleitet werden, oder sind durch einen in einem Wirtsorganismus exprimierten erfindungsgemäßen Vektor in für den Fachmann bekannter Weise herstellbar. Dabei wird typischerweise der Wirtsorganismus zunächst mit dem erfindungsgemäßen Vektor transformiert, der Wirtsorganismus vermehrt und das Polypeptid aus dem Wirtsorganismus, bzw. im Falle einer Sekretion desselben in das Medium, aus diesem gewonnen.

Neben einer direkten Verwendung der erfindungsgemäßen Polypeptide zur Beeinflussung des - zellulären - Geschehens in Schilddrüsengewebe kann dieses auch in der Reinigung, beispielsweise über Affinitätschromatographie, von am zellulären Geschehen beteiligten anderen Komponenten, oder zur Herstellung geeigneter Antikörper verwendet werden, die dann, u.a., ihrerseits zu therapeutischen und/oder diagnostischen Zwecken verwendbar sind.

In Abhängigkeit von den jeweils bestehenden Erfordernissen, wie posttranslationeller Modifikation oder erwünschtem Reinheitsgrad und dergleichen, kann bei Herstellung der erfindungsgemäßen Polypeptide in einem Wirtsorganismus entweder ein prokaryontischer oder ein eukaryontischer Wirtsorganismus von Vorteil sein.

Das erfindungsgemäße Polypeptid kann in geeigneter Weise modifiziert werden. Unter Modifikation soll, unter anderem, eine Fragmentierung, insbesondere Verkürzung, des Moleküls verstanden werden. Modifikation im hierin verwendeten Sinne beinhaltet auch die Markierung des Polypeptids. Letzteres kann mittels sowohl hochmolekularer als auch niedermolekularer Verbindungen erfolgen und schließt die radioaktive, nicht-radioaktive und Fluoreszenzmarkierung ein. Eine Markierung kann auch beispielsweise in Form einer Phosphorylierung oder Glycosylierung des Proteins vorliegen. Entsprechende Markierungsverfahren bzw. Modifikationsverfahren sind in der Technik bekannt (siehe z. B. Protein Methods, 2nd ed., D. M. Bollag et al., Wiley Liss, Inc., New York, NY, 1996).

Unter Modifikation wird erfindungsgemäß auch jede Form von posttranslationaler Modifikation, wie unter Fachleuten bekannt, verstanden, insbesondere proteolytische Verarbeitung des Polypeptids, Anhängen oder Abtrennen von Resten am N-Terminus, Acetylierung des N-Terminus, Myristoylierung des N-Terminus, Anhängen von Glycosyl-Phosphatidyl-Resten, Farnesyl-Resten oder Geranylgeranyl-Resten an den C-Terminus, N-Glycosylierung, O-Glycosylierung, Anhängen von Glycosaminoglycan, Hydroxylierung, Phosphorylierung, ADP-Ribosylierung und Bildung von Disulfidbrücken.

Ebenso ist daran gedacht, daß das erfindungsgemäße Polypeptid durch Aminosäuremutation aus einem erfindungsgemäßen Polypeptid hervorgegangen ist. Unter Aminosäuremutation wird sowohl eine Mutation verstanden, bei der eine Aminosäure durch eine in ihrer Seitenkette ähnliche Aminosäure ausgetauscht wird (konservative Mutation), beispielsweise I zu L oder D zu E, sowie eine Mutation, bei der eine Aminosäure durch eine andere Aminosäure ausgetauscht wird, ohne daß dieser Austausch sich nachteilig auf die Funktion des codierten Polypeptids auswirkt (stille Mutation). Die Funktion des codierten Polypeptids kann durch einen geeigneten Assay überprüft werden. Ein solcher funktionaler Assay für die KRAB-Domäne bzw. die Zinkfinger-Domäne ist z. B. als Bandshift-Assay denkbar. Nützliche funktionale Assays sind in Margolin et al., 1994, Proc. Natl. Acad. Sci. USA 91:4509-4513; Witzgall et al., 1994, Proc. Natl. Acad. Sci. USA 91:4514-4518, Andrews, N.C. and Faller, D.V. 1991, Nucleic Acids Res 19(9): 2499, Elser, B. et al, 1997 J Biol Chem 272(44):27908-12, Fan, C.M. and Maniatis, T., 1989, Embo J 8(1):101-110, Garner, M.M. and Revzin, A. 1981, Nucleic Acids Res 9(13):3047-3060, Kadonaga, J.T. et al, 1987, Cell 51(6):1079-1090, Pierrou, S. et al., 1995, Anal Biochem, 229(1): 99-105, Singh, H. et al, 1986, Nature 319(6049): 154-158, Thiesen, H.J. and Bach, C., 1993, Ann N Y Acad Sci 684:246-249 sowie Vissing et al., 1995, FEBS 369:153-157 offenbart.

Ein erfindungsgemäßes Polypeptid umfaßt eine KRAB-Domäne umfassend die Aminosäuresequenz von SEQ.ID. No.1 und eine Zinkfinger-Domäne mit der Aminosäuresequenz von SEQ.ID. No.2.

Ein erfindungsgemäßes Polypeptid kann insbesondere die in SEQ ID NO: 4 gezeigte Sequenz umfassen.

Mit den erfindungsgemäßen Zellen lassen sich weitergehende Vorteile realisieren. Diese umfassen unter anderem die Herstellung entsprechender Nukleinsäuresequenzen bzw. daraus abgeleiteter Genprodukte.

Insbesondere die Insertion der erfindungsgemäßen Nukleinsäuresequenzen im Genom einer Zelle ist von besonderer Bedeutung, beispielsweise für das weitere Studium des Einflusses derartiger Sequenzen, insbesondere im zellulären Umfeld. Dabei können Gendosiseffekte und dergleichen untersucht bzw. zu diagnostischen und/oder therapeutischen Zwecken ausgenutzt werden. Ganz besonders vorteilhaft scheint dabei ein Zustand, bei dem ausgehend von diploiden Zellen lediglich ein Chromosom die erfindungsgemäße Nukleinsäuresequenz, und bevorzugt in der ihrer Position in Bande 19q13 entsprechenden Position inseriert, enthält und das zweite Chromosom keine Chromosomentranslokation unter Einbeziehung der chromosomalen Bande 19q13 aufweist. Derartige Zellen können beispielsweise als Positiv- und/oder Negativkontrolle in einem diagnostischen Ansatz dienen.

Im Lichte der hierin offenbarten technischen Lehre können sodann auch Antikörper gegen ein Genprodukt der erfindungsgemäßen Nukleinsäuresequenzen, aber auch gegen die Nukleinsäuresequenz selbst, hergestellt werden. Damit ergeben sich auch in diesem Falle die dem Fachmann allgemein aus dem Vorhandensein von Antikörpern gegen chemische Verbindungen bekannten Vorteile. Insbesondere ist damit beispielsweise eine Reinigung der genannten Verbindungen möglich, deren Detektion, aber auch die Beeinflussung der biologischen Aktivität, auch der biologischen Verfügbarkeit, der Verbindungen, auf die der Antikörper gerichtet ist, sowohl in situ, als auch ex vivo bzw. in vitro. Genauer gesagt können insbesondere unter Verwendung der monoklonalen Antikörper die Genprodukte spezifisch detektiert werden bzw. auf zellulärer Ebene eine Interaktion der Genprodukte bzw. Nukleinsäuresequenz mit anderen zellulären Komponenten beeinflußt und somit spezifisch in das zelluläre Geschehen eingegriffen werden. In Abhängigkeit von der Wirkung der jeweiligen Verbindungen, auf die der Antikörper gerichtet ist und auf die er im betrachteten System seine Wirkung entfaltet, können prinzipiell stimulierende wie inhibierende Effekte erzielt werden.

Unter Antikörper werden hierin sowohl polyklonale Antikörper als auch monoklonale Antikörper verstanden. Besonders bevorzugt jedoch sind monoklonale Antikörper aufgrund der erhöhten Spezifität. Es sind jedoch Anwendungsfälle denkbar, in denen zum einen die Reinheit bzw. Spezifität von polyklonalen Antikörpern ausreichend bzw. die Vielzahl der bei polyklonalen Antikörpern realisierten Spezifitäten und anderen Eigenschaften in vorteilhafter Weise genutzt werden können. Die Herstellung u. Verwendung von Antikörpern ist in Antibodies: A Laboratory Manual (E. Harlow & D. Lane, Cold Spring Harbor Laboratory, NY, 1988).

Weiterhin ist es im Rahmen dieser Erfindung, daß der Antikörper auch ein einzelkettiger Antikörper sein kann.

Es ist auch im Rahmen der vorliegenden Erfindung, daß der Antikörper fragmentiert, insbesondere verkürzt ist. Dies schließt ein, daß der Antikörper weitestgehend trunkiert ist, solange noch die Antikörper spezifische Eigenschaft, d.h. Bindung an ein definiertes Epitop, gegeben ist. Trunkierung ist besonders dann von Vorteil, wenn der entsprechende Antikörper auf zellulärer Ebene verwendet werden soll, da er gegenüber einem vollständigen Antikörper verbesserte Permeations- und Diffusionseigenschaften aufweist.

Darüber hinaus sind auch noch andere Formen der Modifikation vorgesehen, die dem Fachmann auf dem Gebiet bekannt sind und beispielsweise beschrieben sind in Antibodies: A Laboratory Manual (E. Harlow & D. Lane, Cold Spring Harbor Laboratory, NY, 1988). Allgemein kann festgehalten werden, daß die Modifizierung von Antikörpern prinzipiell in ähnlicher Weise erfolgen kann, wie die von Polypeptiden und, in bestimmten Umfang, von Nukleinsäuren, und das hierzu oben Gesagte gilt sinngemäß auch für den erfindungsgemäßen Antikörper.

Mit Blick auf eine pharmazeutische Zusammensetzung, welche ein Ribozym neben dem pharmazeutisch akzeptablen Träger umfaßt bzw. die Verwendung des erfindungsgemäßen Ribozyms als Medikament, und insbesondere zur Behandlung von Funktionsstörungen, Hyperplasien und Tumoren der Schilddrüse, ist es auch möglich, daß das Ribozym so konstruiert wird, das es spezifisch auf die erfindungsgemäßen Nukleinsäuresequenzen einwirkt und somit auch hier die Expression bzw. Translation auf zellulärer Ebene kontrolliert, was insbesondere unter therapeutischem aber auch diagnostischem Aspekt von Bedeutung ist. Dabei kann aufgrund der Tatsache, daß Ribozyme sowohl intra- als auch intermolekulare katalytische Wirkungen zeigen, auch vorgesehen sein, daß die erfindungsgemäßen Nukleinsäuresequenzen dergestalt geändert werden, daß Bereiche ihrer selbst durch die Ribozymaktivität des geänderten Bereiches gespalten werden. Auch hierin eröffnet sich eine insbesondere therapeutische Möglichkeit, deren Ausgestaltung dem Fachmann im Lichte der nun vorhandenden Sequenzeninformation möglich ist.

Ribozyme sind, ähnlich den erfindungsgemäßen Nukleinsäuresequenzen selbst, besonders dann von Vorteil, aber nicht nur dann, wenn sie, beispielsweise mittels Gentherapie, in die Effektorzelle eingebracht werden. Jedoch ist auch denkbar, daß entsprechende Modifikationen ex vivo vorgenommen werden und solchermaßen modifizierte Zellen dann zur Reimplantation, entweder allogen oder autogen, zur Verfügung stehen. Die Herstellung und Verwendung von Ribozymen ist in Ribozyme Protocols (Philip C. Turner, Ed., Humana Press, Totowa, NY, 1997) offenbart.

Hinsichtlich der Gestaltung des Kits zur Diagnose und/oder Therapie von Funktionsstörungen, Hyperplasie und Tumoren der Schilddrüse ist anzumerken, daß die genaue Gestaltung eines derartigen Kits, d.h. welche Komponenten explizit darin aufgenommen werden, dem Fachmann auf diesem Gebiet bekannt, und insbesondere möglich sind im Lichte der oben gemachten Ausführungen hinsichtlich der Wirkung bzw. Verwendbarkeit der hierin offenbarten Nukleinsäuresequenzen. Der erfindungsgemäße Kit wird in jedem Fall mindestens eines der erfindungsgemäßen Elemente umfassen, das aus der Gruppe ausgewählt ist, die die Nukleinsäuresequenz, den Vektor, das Polypeptid, die Zelle, den Antikörper und das Ribozym, jeweils in ihrer erfindungsgemäßen Ausprägung, umfaßt. Hinsichtlich der Verwendung der erfindungsgemäßen Zellen ist insbesondere deren Verwendung als Reimplantat bzw. als Positiv- und/oder Negativkontrolle in entsprechenden diagnostischen bzw. therapeutischen Ansätzen möglich.

Bei dem erfindungsgemäßen Verfahren zum Nachweis von Funktionsstörungen und Hyperplasien und/oder Tumoren der Schilddrüse, auch i.S. von Karzinomen und Strumen, ist es möglich, daß das Schilddrüsenmaterial in situ, ex vivo oder in vitro vorliegt Der Begriff "Schilddrüsenmaterial", wie hierin verwendet, bezeichnet Material, insbesondere zelluläres Material der Schilddrüse sowohl in deren normalem als auch pathogenem Zustand. Der Begriff des Schilddrüsenmaterials umfaßt damit auch Material von Schilddrüsen mit einer Funktionsstörung, von Hyperplasien der Schilddrüse, von Tumoren der Schilddrüse, einschließlich Karzinomen und Strumen. Die Bestimmung, ob eine Funktionsstörung, Hyperplasie und/oder Tumor vorliegt, erfolgt letztenende durch Vergleich der Wirkung des eingesetzten erfindungsgemäßen Agens bzw. Agenzien auf das zu untersuchende Schilddrüsenmaterial mit dessen/deren Wirkung auf "normales" Schilddrüsengewebe. Weitere. Ausgestaltungsformen des erfindunsgemäßen Verfahrens ergeben sich für den Fachmann auf der Grundlage der hierin enthaltenen Offenbarung.

Die oben gemachten Ausführungen hinsichtlich der verschiedenen Ausführungsformen sowie den damit realisierbaren Vorteilen gelten sinngemäß auch für die erfindungsgemäßen Medikamente, und insbesondere für solche zur Diagnose und/oder Therapie von Funktionsstörungen, Hyperplasien und/oder Tumoren der Schilddrüse in Form der erfindungsgemäßen Sequenz, bzw. deren Verwendung, in Form des erfindungsgemäßen Vektors, bzw. dessen Verwendung, in Form des erfindungsgemäßen Polypeptids bzw. dessen Verwendung, in Form der erfindungsgemäßen Zelle, bzw. deren Verwendung, in Form des erfindungsgemäßen Antikörpers, bzw. dessen Verwendung und in Form des Ribozyms, bzw. dessen Verwendung, die hierin offenbart werden. Die entsprechenden Medikamente können einen Gehalt an einem oder mehreren der vorgenannten Agenzien aufweisen.

Gleiches gilt auch für die erfindungsgemäßen pharmazeutischen Zusammensetzungen. Dabei ist es möglich, daß eine pharmazeutische Zusammensetzung neben dem pharmazeutisch akzeptablen Träger nur eine der aufgeführten Verbindungen umfaßt. Alternativ ist es auch im Rahmen der Erfindung, daß die pharmazeutische Zusammensetzung mehrere der genannten Verbindungen neben dem pharmazeutisch akzeptablen Träger aufweist.

Unter pharmazeutisch akzeptablem Träger sollen hierin alle dem Fachmann bekannten Träger verstanden werden, die typischerweise in Abhängigkeit von der Applikationsform ausgewählt werden. Ein pharmazeutisch akzeptabler Träger kann, unter anderem, Wasser, Pufferlösungen, alkoholische Lösungen und dergleichen sein.

Hinsichtlich des erfindungsgemäßen Verfahrens zur Herstellung von Primern, sei darauf hingewiesen, daß es sich dabei um solche handelt, die eine spezifische Darstellung der erfindungsgemäßen Nukleinsäuresequenzen, bzw. Teilen davon, erlauben.

Bei dem erfindungsgemäßen Verfahren zur Darstellung einer erfindungsgemäßen Nukleinsäuresequenz können, aus welcher Quelle auch immer, die erfindungsgemäßen Nukleinsäuresequenzen bzw. diesen entsprechende Sequenzen unter Verwendung von erfindungsgemäßen Primern als Primer für eine Polymerasekettenreaktion, in all ihren verschiedenen Ausführungen, verwendet werden. Das Design geeigneter Primer sowie die Durchführung von Polymerase-Kettenreaktionen ist in PCR & PCR 2: A practical approach (M. J. McPherson, P. Quirke und G. R. Taylor, Eds., IRL Press, Oxford, England 1991, & M. J. McPherson und B. D. Hames, Eds., IRL Press, Oxford, England 1995) offenbart.

Weitere Merkmale und Vorteile der Erfindung ergeben sich aus den Figuren, dem Sequenzprotokoll sowie den nachfolgenden Beispielen.

Dabei zeigt:

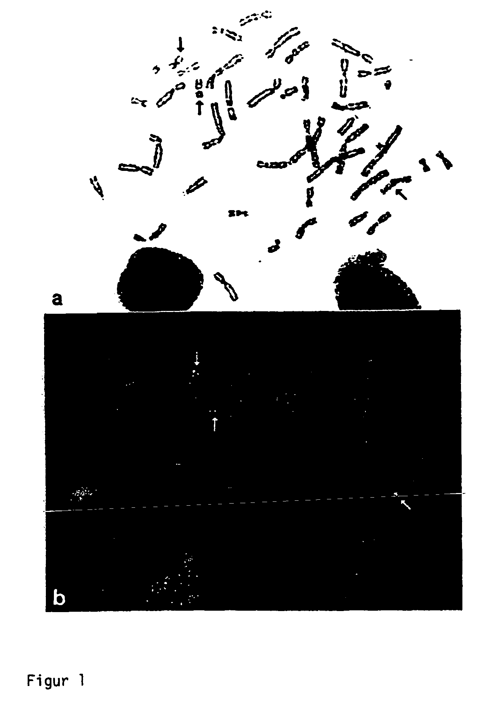

- Figur 1a:: eine Metaphase der Zellinie S-121T/SV40 mit t(5;19)(q13;q13) nach G-Bandenfärbung; Chromosom 19, Derivat-Chromosom 19 (= "der(19)") und Derivat-Chromosom 5 (= "der(5)") sind mit Pfeilen markiert;
- Figur 1b:: dieselbe Metaphase nach FISH mit dem PAC-Klon 19/B; die Hybridisierungssignale sind auf Chromosom 19, der(19) und der(5) lokalisiert;

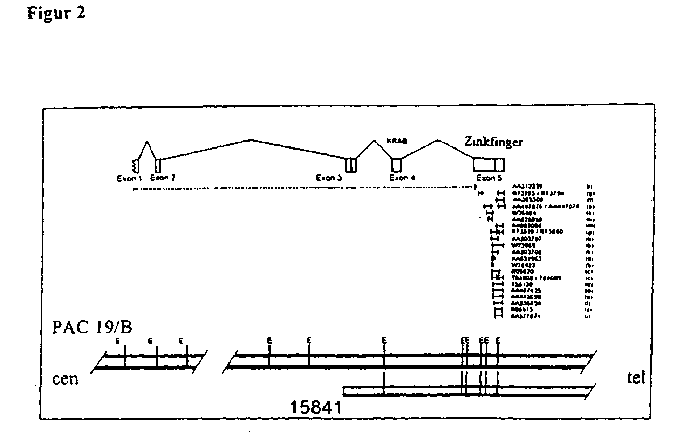

- Figur 2:: die genomische Struktur eines Gens, das im folgenden als RITA bezeichnet (Rearranged in thyroid adenomas), dem Gen, das an spezifischen 19q13-Translokationen in Schilddrüsentumoren beteiligt ist. Graue Boxen repräsentieren die codierenden Regionen, die zu einem offenen Leserahmen von Exon 3 bis Exon 5 gehören, während 5'- oder 3'-nicht-translatierte Regionen als weiße Boxen wiedergegeben sind. Die unter der Exon-Intron-Struktur gezeigten Pfeile repräsentieren cDNA-Klone aus den Datenbanken. Die Pfeilspitzen zeigen die Sequenzierungsrichtung für diese Klone an. Die Quelle der RNA/cDNA ist durch einen einzelnen Buchstaben in Klammern hinter der Genbank-Zugangsnummer jedes Klons angezeigt (a: Gesamtfötus, b: fötales Herz, c: fötale Leber/Milz, d: Lunge, e: Augen-Retina, f: Epiphyse, g: Brust, h: Eierstock-Cortical-Stroma, i: Milchgang-Brusttumore, j: Jurkat-T-Zellen, k: Keimzelltumor, 1: neuroendocrines Lungenkarzinom, m: Testis). Ausgefüllte Balken im unteren Teil der Figur repräsentieren die Position von Teilen von Cosmid 15841 und PAC19/B in Bezug auf RITA; die Abkürzungen "cen" bzw. "tel" betreffen die Lage des Centromers bzw. des Telomers in Bezug auf RITA; die Orientierung des Gens ist von links nach rechts in der Figur telomerwärts.
- Figur 3:: cDNA-Sequenz von RITA, die ebenso die Größe der Exons einschließt, den offenen Leserahmen und die daraus abgeleitete Aminsäuresequenz des offenen Leserahmens. Die Regionen, die der KRAB-Domäne und den Zinkfinger-Motiven (ZF) entsprechen, sind durch schwarz umrandete Balken aufgezeigt;

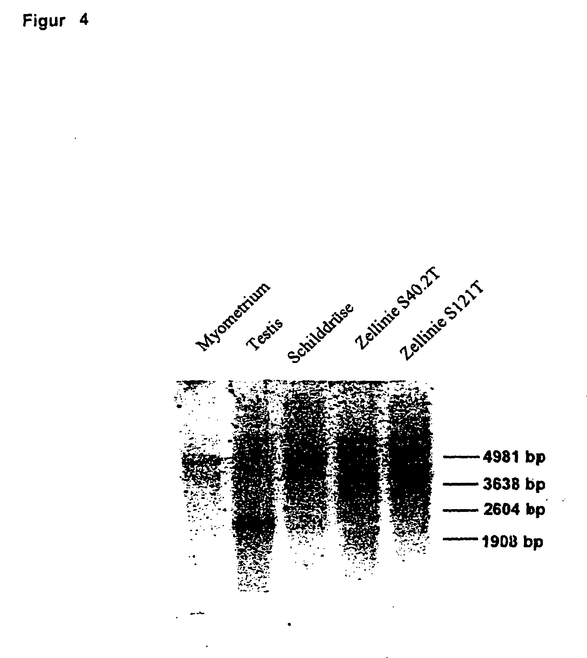

- Figur 4:: Expressionsstudien von RITA: Northernblot-Analyse von Poly(A)+-RNA, die aus Myometrium, Testis, Schilddrüse, Zellinie S40.2T/SV40 und Zellinie S121T/SV40 isoliert worden ist, die zwei Transkripte von ungefähr 4,7 kbp und 5 kbp verschiedener Intensität in Myometrium, Testis und Schilddrüse (Spuren 1-3) offenbart. Ein zusätzliches Transkript von ungefähr 2,1 kbp wird ausschließlich in Testis gefunden (Spur 2). Zellinien S40.2T/SV40 und S121T/SV40 (Spuren 4-5) exprimieren zwei unterschiedliche Transkripte von näherungsweise 5,5 kbp bzw. 6,2 kbp. Auf die Expressionsstudien wird unter anderem in Beispiel 15 eingegangen.

### Beispiel 1

Von Gewebeproben follikulärer Schilddrüsentumoren (n=33), Strumen (n=236) und von normalem, d.h. histologisch unauffälligem Schilddrüsengewebe (n=20) wurden Zellkulturen angesetzt und diese Zellkulturen für eine Chromosomenanalyse aufgearbeitet. Zellkultur und Chromosomenpräparation und -darstellung erfolgten wie bei Belge et al. (Belge, G., Thode, B., Bullerdiek, J., Bartnitzke, S., 1992; Cancer Genet. Cytogenet. 60, 23-26) beschrieben. 4 von 33 Adenomen und 5 von 236 Strumen wiesen Chromosomentranslokationen unter Einbeziehung der chromosomalen Bande 19q13 auf, während sich eine entsprechende Chromosomenveränderung bei keiner Probe des normalen Schilddrüsengewebes fand.

### Beispiel 2

Zellkulturen von Tumoren mit Aberrationen der Bande 19q13 wurden benutzt, um daraus Zellinien für weiterführende Experimente herzustellen. Als Methode dazu wurde ein Teil des SV40 Genoms mit der Calcium-Phosphat-Präzipitationsmethode in die Zellen transfiziert wie von Kazmierczak et al. (Kazmierczak, B., Bartnitzke, S., Hartl, M., Bullerdiek, J., 1990; Cytogenet. Cell Genet. 53, 37-39) beschrieben. Es konnten 3 Zellinien etabliert werden, die jeweils das normale Chromosom 19 und die aus der Translokation resultierenden Derivat-Chromosomen enthielten (Tab. 1).

**Tabelle 1: Übersicht über die von Primärtumoren abgeleiteten Zellinien**

| Lab. Code | Material | Karyotyp |
|---|---|---|
| S-121 | Adenom | 46,XX,t(5;19)(q13;q13) |
| S-141.2 | Struma | 46,XX,t(2;19)(p13;q13) |
| S-40.2 | Struma | 46,XX,t(1;19)(p35 oder p36, 1;q13) |

### Beispiel 3

Zellinien mit t(5;19)(q13;q13) und t(1;19(p35-p36.1;q13) wurden durch Transfektion mit einem Konstrukt erhalten, das das SV40 large-T-Antigen enthält, wie zuvor berichtet (Kazmierczak et al., 1990; Cytogenet Cell Genet 53:37-39); Belge et al., 1992, Cell Biol. Int. Rep., 16:339-347).

### Beispiel 4

Fluoreszenz-in-situ-Hybridisierung (FISH)-Analysen wurden nach GTG-Bandenfärbung derselben Metaphasenpräparationen durchgeführt. Die Behandlung der Metaphasen und darauffolgende FISH-Experimente wurden unter Verwendung des Protokolls von Kievits et al., (1990, Cytogenet. Cell Genet. 53:134-136) durchgeführt. Cosmid- und PAC-DNA wurde mit Biotin-14-dATP durch Nick-Translation markiert (Gibco BRL, Life Technology GmbH, Eggenstein, Deutschland). Für jede Cosmid- oder PAC-Sonde wurden 20 Metaphasen untersucht. Chromosomen wurden mit Propidiumiodid gegengefärbt, auf einem Zeiss-Axiophot-Fluoreszenzmikroskop, unter Verwendung eines FITC-Filters (Zeiss) analysiert und mit dem Power Gene Karyotyping System aufgenommen (PSI, Halladale, U.K.).

### Beispiel 5

Um PAC-Klone zu erhalten, die Cosmid 15841 entsprechen, das aus der Chromosom-19-spezifischen Cosmid-Bibliothek von Lawrence Livermore National Laboratory isoliert worden war (Trask et al., 1992, Genomics 14:162-167; Ashworth et al., 1995, Nat. Genet. 11:422-427), wurde das Cosmid subkloniert und unter Verwendung der Primer M13universal und M13reverse auf einen 373 DNA-Sequencer (Applied Biosystems, Weiterstadt, Deutschland) sequenziert. PAC-Klone wurden dann durch Screenen einer humanen PAC-Bibliothek (Genome Systems, St. Louis, USA) erhalten unter Verwendung eines Satzes Primer, die der Sequenz von Cosmid 15841 entsprachen. PAC19/B wurde in drei Klon-Anordnungen subkloniert. Als erstes wurden *EcoR*I-verdaute DNA-Fragmente in den pGEM11zf(+)-Vektor (Promega, Madison, USA) kloniert. Zwei weitere Klon-Anordnungen wurden hergestellt durch Scherung der DNA, um Zufallsfragmente mit einer durchschnittlichen Größe von 1 kbp bzw. 3 kbp zu produzieren, und durch Klonierung in den pTZ19R-Vektor (MBI Fermentas, Amherst, USA). *EcoR*I*-* und *BamH*I-verdaute DNA-Fragmente von Cosmid 15841 (Trask et al., 1991, Somat. Cell Mol. Genet. 17:117-36)f; Trask et al., 1992, aaO; Ashworth et al., 1995, aaO) wurden ebenso in den pGEM11zf(+)-Vektor kloniert. Sequenzierung wurde mit Standard- und internen Primern durchgeführt unter Verwendung eines ABI 373A-Sequenzers.

### Beispiel 6

Alignment der Sequenzen und PCR-Primem wurde mit dem Lasergene-Softwarepaket (DNAstar, Madison, USA) durchgeführt. Analysen auf Sequenzhomologien wurden unter Verwendung des BLAST-Programms von NCBI durchgeführt (Altschul et al., 1997, Nucl. Acids Res. 25:3389-3402). Zur Genvorhersage und -zusammenbau wurde GENSCAN verwendet (Burge und Karlin, 1997, J. Mol. Biol. 268:78-94).

### Beispiel 7

Gesamt-RNA wurde aus den zwei Zellinien und normalen Erwachsenengeweben unter Verwendung von TRIzol-Reagen isoliert (Gibco BRL, Life Technologies GmbH, Eggenstein, Deutschland), auf der Grundlage des Einschritt-Säure-Phenol-RNA-Isolierungsverfahrens (Chomczynski und Sacchi, 1987, Anal. Biochem. 162:15-159). Poly(A)⁺-RNAs wurden mit Oligotex dC₁₀T₃₀-Adsorption aufgereinigt (QIAGEN, Hilden, Deutschland). Annähernd 5 µg der Poly(A)+-RNAs wurden denaturiert und auf einem 1 %-Agarose/6 % Formaldehydgel fraktioniert und auf eine Hybond-N⁺-Nylonmembran übertragen (Amersham Pharmacia, Freiburg, Deutschland). Als eine molekulare Sonde wurde ein partieller cDNA-Klon (bp 17-1108) des vermuteten Gens (RITA) (Rearranged in thyroid adenomas) verwendet. Die Sonde war mit ³²P markiert unter Verwendung eines Random-Primer-Extensions-Protokolls (Feinberg und Vogelstein, 1983, Anal. Biochem. 132:6-13). Für sowohl Prähybridisierung als auch Hybridisierung wurde die ExpressHyb-Hybridisierungslösung (Clontech Laboratories, Palo Alto, USA) verwendet. Prähybridisierung wurde für 30 Minuten und Hybridisierung für eine Stunde bei 68°C durchgeführt. Die Membranen wurden zweimal für 20 Minuten bei Zimmertemperatur in 2 x SSC/0,05 % SDS und zweimal für 20 Minuten bei 68°C in 0,1 x SSC/0,1 % SDS gewaschen. Signale wurden sichtbar gemacht unter Verwendung eines STORM-Phosphorimager (Molecular Dynamics, Sunnyvale, USA) oder durch Autoradiographie.

### Beispiel 8

Standard-PCR-Amplifizierung wurde in einem Endvolumen von 50 µl durchgeführt. Ungefähr 60 ng Matrize wurde amplifiziert in einer Standard-PCR-Reaktion mit 400 nM jedes Primers, 300 µM dNTPs, 1 x PCR-Puffer (enthaltend 1,5 mM MgCl₂) und 2,5 U AmpliTaq (Perkin Elmer Applied Biosystems, Weiterstadt, Deutschland).

Alu-PCR wurde verwendet, um genomische Sequenzen oder eng überlappende Fragmente (close sequencing contigs) zu erhalten. Genspezifische Primer wurden designed unter Verwendung von Teilsequenzen von RITA und Sequenzen von PAC19/B oder Cosmid 15841-Subklonen. 400 nM des genspezifischen Primers wurden verwendet für eine Alu-PCR-Reaktion zusammen mit 100 nM-Primer TCCCAAAGTGCTGGGATTACAG bzw. CTGCACTCCAGCCTGG. PCR-Produkte wurden auf einem 1,5 % Agarosegel aufgetrennt, die interessierenden Banden ausgeschnitten, aus den Gelschnitten aufgereinigt mit Glass-Bead-Techniken (QIAExII Gel Isolation Kit, QIAGEN, Hilden, Deutschland), in den pCR2.1-Vektor kloniert (Invitrogen, San Diego, USA) und DNA-Sequenzanalyse unterworfen.

### Beispiel 9

Um eine RITA-spezifische cDNA-Sonde, erzeugt durch RT-PCR, zu erhalten, wurde Gesamt-RNA aus normalen Fibroblasten-Primärkulturen unter Verwendung von TRIzol-Reagent (Gibco BRL, Life Technologies GmbH, Eggenstein, Deutschland) isoliert. Gesamt-RNA wurde denaturiert und auf einem 1 % Agarose/6 % Formaldehyd-Gel zur Qualitätskontrolle fraktioniert. 5 µg der Gesamt-RNA wurde revers-transkribiert in einen ersten Strang cDNA mit M-MLV-Reverse Transcriptase (Gibco BRL, Life Technologies, Eggenstein, Deutschland) unter Verwendung von Primer AAGGATCCGTCGACATCT(17) als Start für die Transkription. Extension wurde durchgeführt für 45 Minuten bei 42°C in einem Volumen von 20 µl. 1 µl des Reaktionsgemischs wurde verwendet als Matrize in einer Standard-PCR-Reaktion unter Verwendung von Primern ACGCAACCGCTGTGTCTCC und AGGCCTTCCCACATTCTTGAC zur Amplifizierung von Exon 1 bis Exon 5 von *RITA.* Als Kontrolle für die cDNA-Qualität wurde GAPDH-Expression mit einer Standard-RT-PCR-Reaktion überprüft, unter Verwendung von Primern GGTGAAGACGCCAGTGGACTC und GTGAAGGTCGGAGTCAACG.

### Beispiel 10

Mit Hilfe der Fluoreszenz in situ Hybridisierung (FISH) wurde die Lage von Cosmid-Klonen der Chromosom-19-spezifischen Cosmid Library des Lawrence Livermore National Laboratory, Livermore, Kalifornien, USA, in ihrer Lage relativ zum Bruchpunkt der chromosomalen Translokation bestimmt.

In zwei Zellinien mit den Translokationen t(5;19) (q13;q13) bzw. t(1;19) (p35-p36.1;q13) wurde die Chromosom-19-Bruchstelle mit FISH-Analyse zwischen Cosmid-Klonen 15841 & 29573 lokalisiert. Diese Klone sind aus der Chromosom-19-spezifischen Cosmid-Bibliothek des Lawrence Livermore National Laboratory (Trask et al. 1992, Genomics 14: 162-167; Ashworth et al. 1995, Nat. Genet. 11: 422-427) isoliert.

### Beispiel 11

Aus der unter Beispiel 1 genannten Tumorserie wurden weitere Tumoren mit Aberrationen der chromosomalen Bande 19q13 ausgesucht (Tab. 2). Metaphasenpräparationen dieser Tumoren wurden für die Fluoreszenz in situ Hybridisierung verwendet. Auch hier zeigte sich, daß alle Bruchpunkte innerhalb des zuvor definierten Segmentes lagen.

**Tabelle 2: Übersicht über die in die FISH eingesetzten Primärtumoren**

| | | |
|---|---|---|
| Lab. Code | Material | Karyotyp |
| S-172 | Struma | 46,XX,t(2;19)(p13;q13) |
| S-216 | Adenom | 46,XX,t(10;19)(q24;q13) |

### Beispiel 12

Um PAC-Klone zu erhalten, die die Region der chromosomalen Bruchstellen abdecken, wurde ein Satz Primer designed, der der Sequenz von Cosmid 15841 entspricht. Dieser Satz Primer wurde verwendet, um eine humane PAC-Bibliothek (Genome Systems, St. Louis, USA) zu screenen. Das Screenen führte zu zwei PAC-Klonen, die hierin als PAC19/A bzw. PAC19/B bezeichnet werden. Diese Klone wurden zunächst für FISH-Studien an der Zellinie S-121T/SV40 verwendet, die eine Translokation t(5;19) (q13;q13) zeigte. Mit dem PAC-Klon 19/A wurden Signale auf dem normalen Chromosom 19 und dem der(19)-Chromosom nachgewiesen. Im Gegensatz hierzu zeigt PAC-Klon 19/B Signale auf dem normalen Chromosom 19, auf dem der(19)-Chromosom und auf dem der(5)-Chromosom. Somit überspannt PAC19/B die Bruchstelle dieser Zellinie. Weitere FISH-Studien mit PAC19/B an Zellen der zweiten Zellinie mit Translokation t(1;19) (p35 oder p36.1;q13) offenbarte Hybridisierungssignale, die sich sowohl auf das normale Chromosom 19 als auch auf beide Derivatchromosomen kartieren ließen, was andeutet, daß dieselbe Bruchstellenregion bei dieser Translokation beteiligt war.

Die DNA-Sequenzanalyse von einem der Subklone, die von dem PAC-Klon PAC19/B abgeleitet waren, offenbarte eine Sequenzhomologie mit mehreren cDNA-Klonen aus den Datenbanken. Ein Vergleich von EST-Sequenzen mit der genomischen Sequenz des PAC-Klons ermöglichte die Rekonstruktion der Struktur des Gens, einschließlich der Bestimmung seiner Exon-Intron-Grenzen. Die Gesamtgröße der soweit klonierten cDNA ist 2015 bp, jedoch stellt dies u. U. noch nicht die cDNA in ihrer gesamten Länge dar.

Was die genomische Struktur anbetrifft, hat das Gen 5 Exons. Ein Teil von Exon 5 entspricht der 3'-nicht-translatierten Region, während sein verbleibender Teil eine Krüppel-artige Zinkfingerregion (engl.: "Krüppel-like zinc finger region") codiert (Schuh et al., 1986, Cell, 47: 1025-1032; Bellefroid et al., 1989, DNA, 8: 377-387). Wegen der hohen Homologie von Exon 4 mit Teilen von ZNF 140 (Vissing et al., 1995, FEBS, 369: 153-157) und ZNF 91 (Bellefroid et al., 1993, EMBO J., 12: 1363-1374) kann geschlossen werden, daß dieser Teil des Gens für eine KRAB-A-Domäne codiert (Bellefroid et al., 1991, Proc. Nacl. Acad. Sci., USA, 88: 3608-3612; Rosati et al., 1991, Nucl. Acids. Res., 19: 5661-5667). Exon 3 enthält ein mutmaßliches Initiationscodon, das auf die Konsensussequenz A/GCCATGG paßt (Kozak M. 1987, J. Mol. Biol. 196: 947-950), weshalb Exon 1 - 3 die 5'-nicht-translatierte Region darstellen könnten.

Die Lage der einzelnen Exons ist wie folgt:
- Exon 1:: Nukleotide 1 - 49;
- Exon 2:: Nukleotide 50 - 113;
- Exon 3:: Nukleotide 114 - 195;
- Exon 4:: Nukleotide 196 - 322;
- Exon 5:: Nukleotide 323 - 2014.

Die für RITA codierende Sequenz erstreckt sich von Nukleotid 187 - 1575. Zwischen Nukleotid 1683 und Nukleotid 1689 befindet sich ein Poly(A)-Signal. Die KRAB-Domäne wird durch Nukleotide 196 - 322 codiert.

Hier wird unter Motiv ein Sequenzabschnitt auf DNA und/oder Proteinebene verstanden, der sich unter Abwandlungen oder identisch innerhalb der Sequenz wiederholt. Unter Proteindomäne wird eine Polypeptidkette oder der Teil einer Polypeptidkette verstanden, der sich unabhängig in eine stabile Tertiärstruktur falten kann. Zinkfinger-Motive sind, wie auch in Figur 3 zu sehen ist, bei folgenden Nukleotiden zu finden:
1. Nukleotide 577 - 660;
2. Nukleotide 661 - 744;
3. Nukleotide 745 - 828;
4. Nukleotide 829 - 912;
5. Nukleotide 913 - 996;
6. Nukleotide 997-1080;
7. Nukleotide 1081-1164;
8. Nukleotide 1165 - 1248;
9. Nukleotide 1249 - 1332;
10. Nukleotide 1333 - 1416;
11. Nukleotide 1417 - 1500;
12. Nukleotide 1501 - 1575.

Die genannten Zinkfinger-Motive werden von der Zinkfinger-Domäne umfaßt.

Das in SEQ ID NO: 4 dargestellte Polypeptid, das durch die in SEQ ID NO: 3 dargestellte cDNA codiert wird, hat eine relative Molekülmassee von 53.737,79 Daltons, umfaßt 436 Aminosäuren, von denen 81 K bzw. R sind (basische Aminosäuren), 54 Aminosäuren D bzw. E sind (saure Aminosäuren), 96 A bzw. I bzw. L bzw. F bzw. W bzw. V sind (hydrophobe Aminosäuren) und 144 Aminosäuren N bzw. C bzw. Q bzw. S bzw. T bzw. Y (polare Aminosäuren) sind. Der isoelektrische Punkt beträgt 8,9.

Die transkriptionelle Orientierung des Gens ist in Richtung auf das Telomer, wie aus den folgenden Resultaten abgeleitet werden kann: Mit Hilfe von FISH wurde Cosmid 15841 nur auf das der(19)-Chromosom kartiert, aber in beiden Zellinien wurden Signale von PAC19/B dem der(19)-Chromosom sowie dem der(5)-Chromosom oder dem der(1)-Chromosom zugewiesen. Andererseits konnte mit Hilfe von PCR unter Verwendung von für einzelne Exons spezifischen Primern gezeigt werden, daß Cosmid 15841 nur die Exons 3-5 des Gens enthält, während PAC19B alle soweit identifizierten Exons enthält. Deshalb erfolgt die Überlappung zwischen den beiden Klonen im 3'-Teil des Gens.

Ein typisches Poly(A)-Signal liegt bei bp 1684. Eine alternative Poly(A)-Stelle AAATGAAAA liegt 30 bp stromaufwärts vom 3'-Ende.

Northernblot-Hybridisierung einer Sonde, die für das Gen spezifisch ist (bp 17-1108), auf Poly(A)⁺-Northemblots offenbarte eine Hybridisierung an zwei Transkripte von näherungsweise 4,7 kbp bzw. 5 kbp in normalem Schilddrüsengewebe und in Myometrium. In RNA aus Testis wurde eine zusätzliche starke Bande von 2,1 kbp nachgewiesen. Keines dieser Transkripte konnte in den zwei Zellinien mit 19 q-Aberrationen gefunden werden, die statt dessen zwei unterschiedliche Transkripte von ungefähr 5,5 kbp und 6,2 kbp exprimierten.

### Beispiel 13

Von den oben genannten Zellinien und von in flüssigem Stickstoff konserviertem Material der zwei Primärtumoren mit den 19q13-Aberrationen wurde nach Standardverfahren RNA isoliert und diese RNA für Northem-Blots benutzt. Nach Hybridisierung mit der cDNA des Gens RITA zeigten sich deutliche Banden, die den Schluß zulassen, daß Sequenzen dieses PAC-Klons einem Gen zugehören, das in follikulären Schilddrüsentumoren exprimiert wird.

### Beispiel 14

Mittels der unter den Beispielen 1 und 5 beschriebenen Methoden wurden Chromosomenanalysen und molekular-zytogenetische Untersuchungen an 5 follikulären Schilddrüsencarcinomen durchgeführt. Bei zwei dieser Tumoren zeigten sich bei komplexen Karyotypveränderungen u.a. ebenfalls Translokationen mit Bruchpunkten in 19q13. Die ebenfalls durchgeführte FISH ergab eine Lokalisation der Bruchpunkte innerhalb des von PAC (PAC19/B) abgedeckten Bereiches.

### Beispiel 15

Daß das hierin offenbarte Gen bzw. die hierin offenbarten Nukleinsäuresequenzen das Target-Gen der 19q13-Aberrationen, und insbesondere von 19q13-Translokationen in Schilddrüsentumoren, ist, wird durch drei experimentelle Befunde nahegelegt.
1. Die Bruchstellen sind intragenisch oder nicht mehr als 200 kbp außerhalb der 5'- und 3'-Enden des Gens, was entweder die Bildung eines Fusionsgens oder die transkriptionale Deregulierung des Gens nahelegt.
2. Hinsichtlich des Expressionsmusters des Gens scheint es, daß das Gen in einer Vielzahl normaler Gewebe exprimiert wird wie von Datenbank-Eintragungen und den oben beschriebenen Northernblot-Resultaten gezeigt wird. Nichtsdestotrotz sind in normalem Gewebe drei verschiedene Transkripte gefunden worden, von denen das kleinste nur in testikularem Gewebe nachgewiesen werden konnte, während die zwei größeren Transkripte ubiquitär exprimiert werden. Im Gegensatz ist ein einzigartiges, ungewöhnliches Expressionsmuster für das Gen RITA nur in den zwei Zellinien mit den Translokationen t(5;19)(q13;q13) bzw. t(1;19)(p35-p36.1;q13) nachgewiesen worden.
3. Die Struktur des Gens legt seine Beteiligung an Transkriptionsregulierungen nahe. Sein Genprodukt, das RITA-Protein, gehört zu der Klasse der KRÜPPEL-assoziierten Box(KRAB)-Zinkfinger-Proteinen (Bellefroid et al., 1991, aaO; Rosati et al., 1991, aaO). Diese Proteine bilden eine große Unterfamilie, bei der die KRAB-A-Domäne als eine potente Transkriptionsrepressor-Domäne identifiziert worden ist (Margolin et al., 1994, Proc. Natl. Acad. Sci. USA 91:4509-4513). Es ist geschätzt worden, daß mehrere hundert Gene, die für Krüppel-artige Proteine codieren, im Säugetiergenom existieren (Bellefroid et al., 1991, aaO). Gene, die für KRAB-Zinkfinger-Proteine codieren, machen ungefähr ein Drittel dieser aus. Die Bindung des Transkriptionsrepressor-Proteins Kid-1, einem weiteren KRAB-Zinkfinger-Protein, an eine individuelle DNA-Struktur, ist vor kurzem demonstriert worden (Elser et al., 1997, J. Bio. Chem. 272:279908-27912). Auf den ersten Blick legt die mögliche Funktion von RITA als Transkriptionsregulator die Vermutung nahe, daß die 19q13-Translokationen über einen Verlust an Funktion wirken (loss-of-function). Andererseits legt der Mangel an Deletionen dieser Region in Schilddrüsentumoren sowie die Spezifität einiger dieser Aberrationen einen Zugewinn von Funktion nahe (gain-of-function). Das Wilms'-Tumorgen WT1 codiert für ein klassisches Zinkfinger-Protein (Call et al., 1990, Cell 60:509-520; Gessler et al., 1990, Nature 343:774-778; Haber et al., 1990, Cell 61:1257-1269) und teilt einige gemeinsame Eigenschaften mit den KRAB-Zinkfinger-Proteinen im Hinblick auf seine DNA-Bindungscharakteristik dar (Elser et al., 1997, aaO). Es gibt eine Vielzahl von experimentellen Befunden, daß WT1-Mutationen im Hinblick auf die Entwicklung von Wilms'-Tumoren rezessiv wirken. Im Gegensatz hierzu führt eine spezifische Translokation t(11;22)(p13;q12), die in desmoplastischen Rundzell-Tumoren beobachtet wird, zur Bildung von chimären Transkripten des Gens EWS und WT1 (Ladanyi und Gerald, 1994, Cancer Res. 54:2837-2840; Rauscher et al., 1994, Cold Spring Harb. Symp. Quant. Biol. 59:137-146). In dem daraus resultierenden Protein wird die RNA-Bindungsdomäne von EWS durch das Zinkfinger-Motiv von WT1 ersetzt. Es gibt starke Hinweise darauf, daß dieses Fusionstranskript bei der Entwicklung von desmoplastischen Rundzell-Tumoren essentiell ist, was ein Beispiel dafür bietet, wie das Gen auch durch einen dominanten Zugewinn von Funktion (gain-of-function) beeinträchtigt werden kann.

Obwohl die Ergebnisse der Northernblot-Analysen nahelegen, daß die Struktur von RITA sowie seine Expressionsmuster, vermutlich wegen seiner großen Nähe zur Bruchstellenregion noch nicht vollkommen aufgeklärt sind, wird durch die vorliegende Anmeldung dennoch offenbart, daß RITA ein Target-Gen von spezifischen 19q-Aberrationen in Schilddrüsentumoren ist, das als solches für Diagnose, Prävention und Behandlung von Funktionsstörungen der Schilddrüse, Hyperplasien der Schilddrüse und Tumoren der Schilddrüse genutzt werden kann.

## Patentansprüche

1. Nukleinsäure, die für ein das zelluläre Geschehen in Schilddrüsengewebe beeinflussendes Polypeptid codiert, wobei das Polypeptid eine KRAB-Domäne und mindesten ein Zinkfinger-Motiv umfaßt und die KRAB-Domäne die Aminosäuresequenz von SEQ ID NO: 1 umfaßt und das Zinkfinger-Motiv ein solches ist, wie es in der Zinkfinger-Domäne mit der Aminosäuresequenz von SEQ ID No: 2 umfaßt ist.

2. Nukleinsäure nach Anspruch 1, **dadurch gekennzeichnet, daß** sie die Nukleinsäuresequenz von SEQ ID NO: 3 umfaßt.

3. Vektor, **dadurch gekennzeichnet, daß** er eine Nukleinsäuresequenz nach einem der vorangehenden Ansprüche umfaßt.

4. Vektor nach Anspruch 3, **dadurch gekennzeichnet, daß** er weiterhin mindestens ein Element umfaßt, das ausgewählt ist aus der Gruppe, die Promotoren, Terminatoren und Enhancer umfaßt.

5. Vektor nach Anspruch 3 oder Anspruch 4, **dadurch gekennzeichnet, daß** er ein Expressionsvektor ist.

6. Vektor nach einem der Ansprüche 3 bis 5, **dadurch gekennzeichnet, daß** mindestens ein Promotor im Leserahmen mit mindestens einem für ein Polypeptid codierenden Teil einer Nukleinsäuresequenz nach einem der Ansprüche 1 bis 2 ist.

7. Polypeptid, das das zelluläre Geschehen in Schilddrüsengewebe beeinflußt und eine KRAB-Domäne und eine Zinkfinger-Domäne umfaßt, wobei das Polypeptid durch eine Nukleinsäuresequenz nach einem der Ansprüche 1 bis 2 codiert wird.

8. Polypeptid nach Anspruch 7, **dadurch gekennzeichnet, daß** es modifiziert ist.

9. Zelle, die einen Vektor nach einem der Ansprüche 3 bis 6 umfaßt.

10. Antikörper, **dadurch gekennzeichnet, daß** er gegen ein Polypeptid nach einem der Ansprüche 7 bis 8 gerichtet ist.

11. Antikörper, **dadurch gekennzeichnet, daß** er gegen eine Nukleinsäure nach einem der Ansprüche 1 bis 2 gerichtet ist.

12. Ribozym, **dadurch gekennzeichnet, daß** es gegen eine Nukleinsäure nach einem der Ansprüche 1 bis 2 gerichtet ist.

13. Ribozym nach Anspruch 12, **dadurch gekennzeichnet, daß** es zumindest einen Teil einer Nukleinsäure nach einem der Ansprüche 1 bis 2 umfaßt.

14. Verwendung einer Nukleinsäure nach einem der Ansprüche 1 bis 2 zur Herstellung eines Mittels zur Diagnose und/oder Therapie von Funktionsstörungen der Schilddrüse und/oder Hyperplasien der Schilddrüse und/oder Schilddrüsentumoren.

15. Verwendung einer Nukleinsäure nach einem der Ansprüche 1 bis 2 zur Herstellung eines Medikaments.

16. Verwendung eines Polypeptids nach einem der Ansprüche 7 bis 8 zur Herstellung eines Mittels zur Diagnose und/oder Therapie von Funktionsstörungen der Schilddrüse und/oder Hyperplasien der Schilddrüse und/oder Schilddrüsentumoren.

17. Verwendung eines Polypeptids nach einem der Ansprüche 7 bis 8 zur Herstellung eines Medikaments.

18. Verwendung eines Antikörpers nach einem der Ansprüche 10 bis 11 zur Herstellung eines Mittels zur Diagnose und/oder Therapie von Funktionsstörungen der Schilddrüse und/oder Hyperplasien der Schilddrüse und/oder Schilddrüsentumoren.

19. Verwendung eines Antikörpers nach einem der Ansprüche 10 bis 11 zur Herstellung eines Medikaments.

20. Kit zur Diagnose von Funktionsstörungen der Schilddrüse und/oder Hyperplasien der Schilddrüse und/oder Schilddrüsentumoren, **dadurch gekennzeichnet, daß** der Kit mindestens ein Element umfaßt, das ausgewählt ist aus der Gruppe, die eine Nukleinsäure, einen Vektor, ein Polypeptid, eine Zelle, einen Antikörper und ein Ribozym, jeweils nach einem der vorangehenden Ansprüche, umfaßt.

21. In vitro-Verfahren zum Nachweis von Funktionsstörungen der Schilddrüse und/oder Hyperplasien der Schilddrüse und/oder Tumoren der Schilddrüse, **gekennzeichnet durch** die Schritte:
- Kontaktieren von Schilddrüsenmaterial mit einem Agens, das ausgewählt ist aus der Gruppe, die eine Nukleinsäure, einen Vektor, ein Polypeptid, einen Antikörper, ein Ribozym und eine Zelle, jeweils nach einen der vorangehenden Ansprüche, umfaßt, und
- Bestimmen, ob Funktionsstörungen der Schilddrüse und/oder Hyperplasien der Schilddrüse und/oder Tumore der Schilddrüse vorliegen.

22. Verfahren nach Ansprüch 21, **dadurch gekennzeichnet, daß** das Schilddrüsenmaterial ex vivo vorliegt.

23. Verwendung einer Nukleinsäure nach einem der Ansprüche 1 bis 2 als Primer.

24. Primer zum Darstellen und/oder Screenen und/oder Detektieren einer Nukleinsäure, **dadurch gekennzeichnet, daß** er zu einem Teil einer Nukleinsäuresequenz nach einem der Ansrpüche 1 bis 2 komplementär ist.

25. Verfahren zum Darstellen einer Nukleinsäure, die eine Sequenz umfaßt, welche in Schilddrüsentumoren oder Strumen nachgewiesen werden kann, bei denen eine Translokation mit Bruchstelle in der chromosomalen Bande 19q13 vorliegt, wobei die Sequenz innerhalb der chromosomalen Bande 19q13 liegt, **dadurch gekennzeichnet, daß** das Verfahren die Schritte aufweist:
- Bereitstellen von Primern nach Anspruch 24, zum Durchführen einer Polymerase-Kettenreaktion,
- Bereitstellen einer der Bande 19q13 des humanen Chromosoms 19 entnommenen Nukleinsäuresequenz oder einer Nukleinsäure nach einem der Ansprüche 1 bis 2,
- Mischen der Nukleinsäuresequenz bzw. der Nukleinsäure mit den Primern,
- Durchführen einer Polymerase-Kettenreaktion.

26. Pharmazeutische Zusammensetzung, **dadurch gekennzeichnet, daß** sie umfaßt:
mindestens ein Agens, das ausgewählt ist aus der Gruppe, die eine Nukleinsäure, einen Vektor, ein Polypeptid, eine Zelle, einen Antikörper, ein Ribozym, jeweils gemäß einem der vorangehenden Ansprüche, sowie Kombinationen davon umfaßt, und mindestens einen pharmazeutisch akzeptablen Träger.

27. Verwendung einer Nukleinsäure, die mit einer Nukleinsäure nach Anspruch 1 oder 2 hybridisieren kann, zur Herstellung eines Mittels zur Diagnose und/oder Therapie von Funktionsstörungen der Schilddrüse und/oder Hyperplasien der Schilddrüse und/oder Schilddrüsentumoren.

28. Verwendung einer Nukleinsäure, die mit einer Nukleinsäure nach Anspruch 1 oder 2 hybridisieren kann, zur Herstellung eines Medikamentes zur Behandlung von Funktionsstörungen, Hyperplasien und/oder Tumoren der Schilddrüse.

## Claims

1. A nucleic acid which codes for a polypeptide influencing the cellular events in thyroid tissue, wherein the polypeptide comprises a KRAB domain and at least one zinc finger motif, and whereby the KRAB domain comprises the amino acid sequence of SEQ ID NO: 1, and the zinc finger motif is one which is comprised by the zinc finger domain having the amino acid sequence of SEQ ID NO: 2.

2. The nucleic acid according to claim 1, **characterized in that** it comprises the nucleic acid sequence of SEQ ID NO:3.

3. A vector, **characterized in that** it comprises a nucleic acid sequence according to any of the preceding claims.

4. The vector according to claim 3, **characterized in that** it additionally comprises at least one element selected from the group comprising promoters, terminators and enhancers.

5. The vector according to claim 3 or claim 4, **characterized in that** it is an expression vector.

6. The vector according to any of claims 3 to 5, **characterized in that** at least one promoter is in-frame with at least a part of a nucleic acid sequence as claimed in one of the claims 1 to 2 that codes for a polypeptide.

7. A polypeptide influencing the cellular events in thyroid tissue and comprising a KRAB domain and a zinc finger domain, wherein the polypeptide is encoded by a nucleic acid sequence according to any of claims 1 to 2.

8. The polypeptide according to claim 7, **characterized in that** it is modified.

9. A cell comprising a vector according to any of claims 3 to 6.

10. An antibody, **characterized in that** it is directed against a polypeptide according to any of claims 7 to 8.

11. An antibody, **characterized in that** it is directed against a nucleic acid according to any of claims 1 to 2.

12. A ribozyme, **characterized in that** it is directed against a nucleic acid according to any of claims 1 to 2.

13. The ribozyme according to claim 12, **characterized in that** it comprises at least a part of a nucleic acid according to any of claims 1 to 2.

14. Use of a nucleic acid according to any of claims 1 to 2 for the manufacture of an agent for the diagnosis and/or therapy of functional disorders of the thyroid and/or hyperplasiae of the thyroid and/or thyroid tumours.

15. Use of a nucleic acid according to any of claims 1 to 2 for the manufacture of a medicament.

16. Use of a polypeptide according to any of claims 7 to 8 for the manufacture of an agent for the diagnosis and/or therapy of functional disorders of the thyroid and/or hyperplasiae of the thyroid and/or thyroid tumours.

17. Use of a polypeptide according to any of claims 7 to 8 for the manufacture of a medicament.

18. Use of an antibody according to any of claims 10 to 11 for the manufacture of an agent for the diagnosis and/or therapy of functional disorders of the thyroid and/or hyperplasiae of the thyroid and/or thyroid tumours.

19. Use of an antibody according to any of claims 10 to 11 for the manufacture of a medicament.

20. A kit for the diagnosis of functional disorders of the thyroid and/or hyperplasiae of the thyroid and/or thyroid tumours, **characterized in that** the kit comprises at least one element selected from the group comprising a nucleic acid, a vector, a polypeptide, a cell, an antibody and a ribozyme, each according to one of the preceding claims.

21. An *in vitro* method for the detection of functional disorders of the thyroid and/or hyperplasiae of the thyroid and/or tumours of the thyroid, **characterized by** the steps:
- contacting thyroid material with an agent selected from the group comprising a nucleic acid, a vector, a polypeptide, an antibody, a ribozyme and a cell, each according to one of the preceding claims, and
- determining whether functional disorders of the thyroid and/or hyperplasiae of the thyroid and/or tumours of the thyroid are present.

22. The method according to claim 21, **characterized in that** the thyroid material is present *ex vivo.*

23. Use of a nucleic acid according to any of claims 1 to 2 as a primer.

24. A primer for preparing and/or screening and/or detecting a nucleic acid, **characterized in that** it is complementary to a part of a nucleic acid sequence according to any of claims 1 to 2.

25. A method for preparing a nucleic acid comprising a sequence that can be detected in thyroid tumours or strumae, in which a translocation with a breakage point in chromosomal band 19q13 is present, whereby said sequence is located within the chromosomal band 19q13, **characterized in that** the method comprises the steps:
- providing primers according to claim 24, for performing a polymerase chain reaction,
- providing a nucleic acid sequence taken from band 19q13 of human chromosome 19, or a nucleic acid according to any of claims 1 to 2,
- mixing said nucleic acid sequence and nucleic acid, respectively, with the primers,
- performing a polymerase chain reaction.

26. A pharmaceutical composition, **characterized in that** it comprises:
at least one agent selected from the group comprising a nucleic acid, a vector, a polypeptide, a cell, an antibody, a ribozyme, each according to one of the preceding claims, or combinations thereof, and at least one pharmaceutically acceptable carrier.

27. Use of a nucleic acid, which is able to hybridise to a nucleic acid according to claim 1 or 2, for the manufacture of an agent for the diagnosis and/or therapy of functional disorders of the thyroid and/or hyperplasiae of the thyroid and/or thyroid tumours.

28. Use of a nucleic acid, which is able to hybridise to a nucleic acid according to claim 1 or 2, for the manufacture of a medicament for the treatment of functional disorders, hyperplasiae and/or tumours of the thyroid.

## Revendications

1. Acide nucléique qui code pour un polypeptide influençant le déroulement cellulaire dans le tissu thyroïdien, le polypeptide comprenant un domaine KRAB et au moins un motif en doigt de zinc, et le domaine KRAB comprend la séquence d'acides aminés de la SEQ ID n°1 et le motif en doigt de zinc est tel qu'il est contenu dans le domaine en doigt de zinc selon la séquence d'acides aminés de la SEQ ID n°2.

2. Acide nucléique selon la revendication 1, **caractérisé en ce qu'**il comprend la séquence d'acides nucléiques de la SEQ ID n°3.

3. Vecteur **caractérisé en ce qu'**il comprend une séquence d'acides nucléiques selon l'une quelconque des revendications précédentes.

4. Vecteur selon la revendication 3, **caractérisé en ce qu'**il comprend en outre au moins un élément, qui est choisi dans le groupe comprenant les promoteurs, terminateurs et amplificateurs.

5. Vecteur selon la revendication 3 ou 4, **caractérisé en ce qu'**il est un vecteur d'expression.

6. Vecteur selon l'une des revendications 3 à 5, **caractérisé en ce qu'**il s'agit au moins d'un promoteur du cadre de lecture avec au moins une partie codant pour un polypeptide d'une séquence d'acides nucléiques selon l'une des revendications 1 à 2.

7. Polypeptide qui influence le déroulement cellulaire dans le tissu thyroïdien et comprend un domaine KRAB et un domaine en doigt de zinc, le polypeptide étant codé par une séquence d'acides nucléiques selon l'une des revendications 1 à 2.

8. Polypeptide selon la revendication 7, **caractérisé en ce qu'**il est modifié.

9. Cellule, qui comprend un vecteur selon l'une des revendications 3 à 6.

10. Anticorps, **caractérisé en ce qu'**il est dirigé contre un polypeptide selon l'une des revendications 7 à 8.

11. Anticorps, **caractérisé en ce qu'**il est dirigé contre un acide nucléique selon l'une des revendications 1 à 2.

12. Ribozyme, **caractérisé en ce qu'**il est dirigé contre un acide nucléique selon l'une des revendications 1 à 2.

13. Ribozyme selon la revendication 12, **caractérisé en ce qu'**il comprend au moins une partie d'un acide nucléique selon l'une des revendications 1 à 2.

14. Utilisation d'un acide nucléique selon l'une des revendications 1 à 2 pour la fabrication d'un moyen de diagnostic et/ou de traitement de troubles fonctionnels de la thyroïde et/ou de l'hyperplasie de la thyroïde et/ou de tumeurs de la thyroïde.

15. Utilisation d'un acide nucléique selon l'une des revendications 1 à 2 pour la fabrication d'un médicament.

16. Utilisation d'un polypeptide selon l'une des revendications 7 à 8 pour la fabrication d'un moyen de diagnostic et/ou de traitement de troubles fonctionnels de la thyroïde et/ou de l'hyperplasie de la thyroïde et/ou de tumeurs de la thyroïde.

17. Utilisation d'un polypeptide selon l'une des revendications 7 à 8 pour la fabrication d'un médicament.

18. Utilisation d'un anticorps selon l'une des revendications 10 à 11 pour la fabrication d'un moyen de diagnostic et/ou de traitement de troubles fonctionnels de la thyroïde et/ou de l'hyperplasie de la thyroïde et/ou de tumeurs de la thyroïde.

19. Utilisation d'un anticorps selon l'une des revendications 10 à 11 pour la fabrication d'un médicament.

20. Kit de diagnostic de troubles fonctionnels de la thyroïde et/ou de l'hyperplasie de la thyroïde et/ou de tumeurs de la thyroïde, **caractérisé en ce que** le kit comprend au moins un élément, qui est choisi dans le groupe qui comprend un acide nucléique, un vecteur, un polypeptide, une cellule, un anticorps et un ribozyme, respectivement selon l'une des revendications précédentes.

21. Procédé *in vitro* pour la détection de troubles fonctionnels de la thyroïde et/ou de l'hyperplasie de la thyroïde et/ou de tumeurs de la thyroïde, **caractérisé par** les étapes suivantes :
- mettre en contact de la matière thyroïdienne avec un agent qui est choisi dans le groupe comprenant un acide nucléique, un vecteur, un polypeptide, un anticorps, un ribozyme et une cellule, respectivement selon l'une des revendications précédentes, et
- déterminer si des troubles fonctionnels de la thyroïde et/ou de l'hyperplasie de la thyroïde et/ou de tumeurs de la thyroïde existent.

22. Procédé selon la revendication 21, **caractérisé en ce que** la matière thyroïdienne est présente *ex vivo.*

23. Utilisation d'un acide nucléique selon l'une des revendications 1 à 2 comme amorce.

24. Amorce pour la révélation et/ou le dépistage et/ou la détection d'un acide nucléique, **caractérisé en ce qu'**elle est complémentaire d'une partie d'une séquence d'acides nucléiques selon l'une des revendications 1 à 2.

25. Procédé de révélation d'un acide nucléique, qui comprend une séquence, laquelle peut être détectée dans des tumeurs de la thyroïde ou des goitres, dans lesquels existe une translocation avec des sites de césure dans la bande chromosomique 19q13, la séquence présente au sein de la bande chromosomique 19q13 étant **caractérisé en ce que** le procédé présente les étapes suivantes :
- la mise à disposition d'amorces selon la revendication 24, pour la réalisation d'une réaction en chaîne par polymérase,
- la mise à disposition d'une séquence d'acides nucléiques tirée de la bande 19q13 du chromosome humain 19 ou d'un acide nucléique selon l'une des revendications 1 à 2,
- le mélange de la séquence d'acides nucléiques ou de l'acide nucléique avec les amorces,
- la réalisation d'une réaction en chaîne par polymérase.

26. Composition pharmaceutique **caractérisée en ce qu'**elle comprend :
au moins un agent, qui est choisi dans le groupe qui comprend un acide nucléique, un vecteur, un polypeptide, une cellule, un anticorps, un ribozyme, respectivement selon l'une des revendications précédentes, ainsi que leurs combinaisons, et au moins un véhicule pharmaceutiquement acceptable.

27. Utilisation d'un acide nucléique, qui peut s'hybrider avec un acide nucléique selon la revendication 1 ou 2, pour la fabrication d'un agent de diagnostic et/ou de traitement de troubles fonctionnels de la thyroïde et/ou de l'hyperplasie de la thyroïde et/ou de tumeurs de la thyroïde.

28. Utilisation d'un acide nucléique, qui peut s'hybrider avec un acide nucléique selon la revendication 1 ou 2, pour la fabrication d'un médicament pour le traitement de troubles fonctionnels de la thyroïde et/ou de l'hyperplasie de la thyroïde et/ou de tumeurs de la thyroïde.


### SEQUENZPROTOKOLL

<110> Universität Bremen
<120> Nukleinsäuresequenzen von Hyperplasien und Tumoren der Schilddrüse
<130> B 10002 PCT
<140>
   <141>
<160> 4
<170> Patent In Ver. 2.1
<210> 1
   <211> 42
   <212> PRT
   <213> Homo sapiens
<220>
   <221> DOMAIN
   <222> (1)..(42)
   <223> KRAB-Domäne
<220>
   <223> Bemerkung: Die hier als 1-42 bezeichneten Reste entsprechen den Resten 4-45 des von RITA codierten Genproduktes bzw. den Resten 4-45 der unter SEQ ID NO:4 aufgeführten Sequenz.
<400> 1
   Gly Leu Val Thr Phe Ala Asp Val Ala Ile Asp Phe Ser Gln Glu Glu
    1               5                  10                  15
   Trp Ala Cys Leu Asn Ser Ala Gln Arg Asp Leu Tyr Trp Asp Val Met
               20                  25                  30
   Leu Glu Asn Tyr Ser Asn Leu Val Ser Leu
           35                  40
<210> 2
   <211> 333
   <212> PRT
   <213> Homo sapiens
<220>
   <221> DOMAIN
   <222> (1)..(333)
   <223> Zinkfinger-Domäne
<220>
   <223> Bemerkung: Die hier als 1-333 bezeichneten Reste entsprechen Resten 131-463 des von RITA codierten Genproduktes bzw. der unter SEQ ID NO:4 aufgeführten Sequenz.
<400> 2
   Phe Glu Cys Lys Asp Cys Gly Lys Ala Phe Ser Arg Gly Tyr Gln Leu
    1               5                   10                  15
   Ser Gln His Gln Lys Ile His Thr Gly Glu Lys Pro Tyr Glu Cys Lys
                   20                  25                  30
   Glu Cys Lys Lys Ala Phe Arg Trp Gly Asn Gln Leu Thr Gln His Gln
               35                  40                  45
   Lys Ile His Thr Gly Glu Lys Pro Tyr Glu Cys Lys Asp Cys Gly Lys
       50                  55                  60
   Ala Phe Arg Trp Gly Ser Ser Leu Val Ile His Lys Arg Ile His Thr
   65                  70                  75                  80
   Gly Glu Lys Pro Tyr Glu Cys Lys Asp Cys Gly Lys Ala Phe Arg Arg
                   85                  90                  95
   Gly Asp Glu Leu Thr Gln His Gln Arg Phe His Thr Gly Glu Lys Asp
               100                 105                 110
   Tyr Glu Cys Lys Asp Cys Gly Lys Thr Phe Ser Arg Val Tyr Lys Leu
           115                 120                 125
   Ile Gln His Lys Arg Ile His Ser Gly Glu Lys Pro Tyr Glu Cys Lys
       130                 135                 140
   Asp Cys Gly Lys Ala Phe Ile Cys Gly Ser Ser Leu Ile Gln His Lys
   145                 150                 155                 160
   Arg Ile His Thr Gly Glu Lys Pro Tyr Glu Cys Gln Glu Cys Gly Lys
                   165                 170                 175
   Ala Phe Thr Arg Val Asn Tyr Leu Thr Gln His Gln Lys Ile His Thr
               180                 185                 190
   Gly Glu Lys Pro His Glu Cys Lys Glu Cys Gly Lys Ala Phe Arg Trp
           195                 200                 205
   Gly Ser Ser Leu Val Lys His Glu Arg Ile His Thr Gly Glu Lys Pro
       210                 215                 220
   Tyr Lys Cys Thr Glu Cys Gly Lys Ala Phe Asn Cys Gly Tyr His Leu
   225                 230                 235                 240
   Thr Gln His Glu Arg Ile His Thr Gly Glu Thr Pro Tyr Lys Cys Lys
                   245                 250                 255
   Glu Cys Gly Lys Ala Phe Ile Tyr Gly Ser Ser Leu Val Lys His Glu
               260                 265                 270
   Arg Ile His Thr Gly Val Lys Pro Tyr Gly Cys Thr Glu Cys Gly Lys
           275                 280                 285
   Ser Phe Ser His Gly His Gln Leu Thr Gln His Gln Lys Thr His Ser
       290                 295                 300
   Gly Ala Lys Ser Tyr Glu Cys Lys Glu Cys Gly Lys Ala Cys Asn His
   305                 310                 315                 320
   Leu Asn His Leu Arg Glu His Gln Arg Ile His Asn Ser
                   325                 330
<210> 3
   <211> 2015
   <212> DNA
   <213> Homo sapiens
<220>
   <221> CDS
   <222> (187)..(1575)
   <223> codierende Region für RITA
<400> 3
   cgcttctgca ccggtgacgc aaccgctgtg tctccgccag tccgcgcagg ccagcatcct 60
   tcagaaaaag catccccgag gaggaagacg aatcgttaaa catcttaggt cagctctagc 120
   ctctcggaat ttgtcttctt cagtggaaac cccgagaaga ctgatcagtt cttcagttct 180
   aaaaca atg gcc cag ggt ttg gtg acg ttc gcc gac gta gcc ata gac    228
          Met Ala Gln Gly Leu Val Thr Phe Ala Asp Val Ala Ile Asp
            1               5                  10
   ttt tct cag gag gag tgg gcc tgt ctg aac tct gct cag agg gac ctg   276
   Phe Ser Gln Glu Glu Trp Ala Cys Leu Asn Ser Ala Gln Arg Asp Leu
    15                  20                  25                  30
   tac tgg gac gtg atg ctg gag aac tac agt aac ttg gtc tca ctg gat   324
   Tyr Trp Asp Val Met Leu Glu Asn Tyr Ser Asn Leu Val Ser Leu Asp
                    35                  40                  45
   ttg gag tca gca tat gaa aat aag agt tta cct aca gaa aaa aac att   372
   Leu Glu Ser Ala Tyr Glu Asn Lys Ser Leu Pro Thr Glu Lys Asn Ile
                50                  55                  60
   cat gaa ata agg gct tcc aaa agg aat tca gat aga aga agt aaa tcc   420
   His Glu Ile Arg Ala Ser Lys Arg Asn Ser Asp Arg Arg Ser Lys Ser
            65                  70                  75
   ctt ggc cgt aac tgg ata tgt gaa ggt acg ctt gaa aga cca cag cgc   468
   Leu Gly Arg Asn Trp Ile Cys Glu Gly Thr Leu Glu Arg Pro Gln Arg
        80                  85                  90
   tcc aga ggg agg tat gtc aat cag atg atc atc aat tat gtc aaa agg   516
   Ser Arg Gly Arg Tyr Val Asn Gln Met Ile Ile Asn Tyr Val Lys Arg
    95                 100                 105                 110
   cct gct act aga gaa ggc acc cct cct aga aca cat cag aga cat cat   564
   Pro Ala Thr Arg Glu Gly Thr Pro Pro Arg Thr His Gln Arg His His
                   115                 120                 125
   aag gag aat tcc ttt gaa tgt aag gac tgt ggg aag gcc ttt agt cgt   612
   Lys Glu Asn Ser Phe Glu Cys Lys Asp Cys Gly Lys Ala Phe Ser Arg
               130                 135                 140
   ggc tat caa ctt agt caa cat cag aaa atc cat act ggt gag aaa cct   660
   Gly Tyr Gln Leu Ser Gln His Gln Lys Ile His Thr Gly Glu Lys Pro
           145                 150                 155
   tat gaa tgt aaa gaa tgt aag aag gcc ttc cgt tgg ggc aat cag ctt   708
   Tyr Glu Cys Lys Glu Cys Lys Lys Ala Phe Arg Trp Gly Asn Gln Leu
       160                 165                 170
   act caa cat caa aaa att cat act ggg gag aag ccc tac gaa tgt aaa   756
   Thr Gln His Gln Lys Ile His Thr Gly Glu Lys Pro Tyr Glu Cys Lys
   175                 180                 185                 190
   gac tgt ggg aag gct ttt cga tgg ggc tca agc ctc gtt att cat aag    804
   Asp Cys Gly Lys Ala Phe Arg Trp Gly Ser Ser Leu Val Ile His Lys
                   195                 200                 205
   agg att cat act ggt gaa aaa ccc tat gaa tgt aaa gac tgt gga aag    852
   Arg Ile His Thr Gly Glu Lys Pro Tyr Glu Cys Lys Asp Cys Gly Lys
               210                 215                 220
   gcc ttt cgg cgt ggt gat gag ctc act cag cac cag aga ttc cac act    900
   Ala Phe Arg Arg Gly Asp Glu Leu Thr Gln His Gln Arg Phe His Thr
           225                 230                 235
   ggg gag aaa gac tac gaa tgc aaa gac tgt ggg aag acc ttt agc cgt    948
   Gly Glu Lys Asp Tyr Glu Cys Lys Asp Cys Gly Lys Thr Phe Ser Arg
       240                 245                 250
   gtg tat aaa ctt att cag cac aag aga att cat agt ggg gag aag cct    996
   Val Tyr Lys Leu Ile Gln His Lys Arg Ile His Ser Gly Glu Lys Pro
   255                 260                 265                 270
   tac gag tgt aaa gac tgt ggg aag gct ttt att tgt ggt tca agc ctc    1044
   Tyr Glu Cys Lys Asp Cys Gly Lys Ala Phe Ile Cys Gly Ser Ser Leu
                   275                 280                 285
   att cag cat aaa aga att cac aca ggt gag aaa ccc tat gaa tgt caa    1092
   Ile Gln His Lys Arg Ile His Thr Gly Glu Lys Pro Tyr Glu Cys Gln
               290                 295                 300
   gaa tgt ggg aag gcc ttt act cga gtc aat tac ctt act cag cat cag    1140
   Glu Cys Gly Lys Ala Phe Thr Arg Val Asn Tyr Leu Thr Gln His Gln
           305                 310                 315
   aag atc cac acc ggt gag aag cct cac gaa tgt aag gag tgt ggg aag    1188
   Lys Ile His Thr Gly Glu Lys Pro His Glu Cys Lys Glu Cys Gly Lys
       320                 325                 330
   gcc ttt cgc tgg ggt tcg agc ctc gtt aag cac gag agg ata cat acg    1236
   Ala Phe Arg Trp Gly Ser Ser Leu Val Lys His Glu Arg Ile His Thr
   335                 340                 345                 350
   ggc gag aag ccg tac aag tgc aca gaa tgt ggg aag gcc ttc aat tgt    1284
   Gly Glu Lys Pro Tyr Lys Cys Thr Glu Cys Gly Lys Ala Phe Asn Cys
                   355                 360                 365
   ggc tat cac ctc act cag cac gag aga atc cac aca ggc gaa acc ccg    1332
   Gly Tyr His Leu Thr Gln His Glu Arg Ile His Thr Gly Glu Thr Pro
               370                 375                 380
   tat aaa tgt aag gag tgt ggg aag gct ttc att tat gga tcg agc ctc    1380
   Tyr Lys Cys Lys Glu Cys Gly Lys Ala Phe Ile Tyr Gly Ser Ser Leu
           385                 390                 395
   gtg aaa cat gag aga att cat acc ggg gtg aaa ccc tat ggg tgt aca    1428
   Val Lys His Glu Arg Ile His Thr Gly Val Lys Pro Tyr Gly Cys Thr
       400                 405                 410
   gaa tgt ggg aag agc ttt agt cac ggc cat cag ctt aca caa cat cag    1476
   Glu Cys Gly Lys Ser Phe Ser His Gly His Gln Leu Thr Gln His Gln
   415                 420                 425                 430
   aaa acg cac agt ggg gcg aaa tcc tac gaa tgt aag gag tgc ggg aag    1524
   Lys Thr His Ser Gly Ala Lys Ser Tyr Glu Cys Lys Glu Cys Gly Lys
                   435                 440                 445
   gca tgt aac cac cta aac cat ctc cga gaa cat cag agg atc cac aac    1572
   Ala Cys Asn His Leu Asn His Leu Arg Glu His Gln Arg Ile His Asn
               450                 455                 460
   agt tgaagagcct tttgaacgca gtagcccgct cgtatctatg gtttcgcttt        1625
   Ser
   ccacagtttg ttacctgcag tcaactgcag ttcaaaaata ttaaatggaa aattccagaa 1685
   ataaagaatt ttaagtctca aatggtgtgc ccttctgagt agcgtgatga aatctctcgc 1745
   tgtccggctc cagccggccg gggatgtgag tcatcccttg gtccagcaca tccacgctgt 1805
   atacgccacc caccctgcta gtgacttagt agccgtcttg gtgatcagat caactatccc 1865
   agcatcacag tgcctgtgcc caagtagtcc tcactttgct taacagtggc cccagagagc 1925
   aggagtagtg atgctggtga ttcggatatg ccaaagagaa gccacaaagt gcttcctttt 1985
   aaatgaaaag gtgaaagttc tcaacttaaa                                  2015
<210> 4
   <211> 463
   <212> PRT
   <213> Homo sapiens
<400> 4
   Met Ala Gln Gly Leu Val Thr Phe Ala Asp Val Ala Ile Asp Phe Ser
   1 5 10 15
   Gln Glu Glu Trp Ala Cys Leu Asn Ser Ala Gln Arg Asp Leu Tyr Trp
   20 25 30
   Asp Val Met Leu Glu Asn Tyr Ser Asn Leu Val Ser Leu Asp Leu Glu
   35 40 45
   Ser Ala Tyr Glu Asn Lys Ser Leu Pro Thr Glu Lys Asn Ile His Glu
   50 55 60
   Ile Arg Ala Ser Lys Arg Asn Ser Asp Arg Arg Ser Lys Ser Leu Gly
   65 70 75 80
   Arg Asn Trp Ile Cys Glu Gly Thr Leu Glu Arg Pro Gln Arg Ser Arg
   85 90 95
   Gly Arg Tyr Val Asn Gln Met Ile Ile Asn Tyr Val Lys Arg Pro Ala
   100 105 110
   Thr Arg Glu Gly Thr Pro Pro Arg Thr His Gln Arg His His Lys Glu
   115 120 125
   Asn Ser Phe Glu Cys Lys Asp Cys Gly Lys Ala Phe Ser Arg Gly Tyr
   130 135 140
   Gln Leu Ser Gln His Gln Lys Ile His Thr Gly Glu Lys Pro Tyr Glu
   145 150 155 160
   Cys Lys Glu Cys Lys Lys Ala Phe Arg Trp Gly Asn Gln Leu Thr Gln
   165 170 175
   His Gln Lys Ile His Thr Gly Glu Lys Pro Tyr Glu Cys Lys Asp Cys
   180 185 190
   Gly Lys Ala Phe Arg Trp Gly Ser Ser Leu Val Ile His Lys Arg Ile
   195 200 205
   His Thr Gly Glu Lys Pro Tyr Glu Cys Lys Asp Cys Gly Lys Ala Phe
   210 215 220
   Arg Arg Gly Asp Glu Leu Thr Gln His Gln Arg Phe His Thr Gly Glu
   225 230 235 240
   Lys Asp Tyr Glu Cys Lys Asp Cys Gly Lys Thr Phe Ser Arg Val Tyr
   245 250 255
   Lys Leu Ile Gln His Lys Arg Ile His Ser Gly Glu Lys Pro Tyr Glu
   260 265 270
   Cys Lys Asp Cys Gly Lys Ala Phe Ile Cys Gly Ser Ser Leu Ile Gln
   275 280 285
   His Lys Arg Ile His Thr Gly Glu Lys Pro Tyr Glu Cys Gln Glu Cys
   290 295 300
   Gly Lys Ala Phe Thr Arg Val Asn Tyr Leu Thr Gln His Gln Lys Ile
   305 310 315 320
   His Thr Gly Glu Lys Pro His Glu Cys Lys Glu Cys Gly Lys Ala Phe
   325 330 335
   Arg Trp Gly Ser Ser Leu Val Lys His Glu Arg Ile His Thr Gly Glu
   340 345 350
   Lys Pro Tyr Lys Cys Thr Glu Cys Gly Lys Ala Phe Asn Cys Gly Tyr
   355 360 365
   His Leu Thr Gln His Glu Arg Ile His Thr Gly Glu Thr Pro Tyr Lys
   370 375 380
   Cys Lys Glu Cys Gly Lys Ala Phe Ile Tyr Gly Ser Ser Leu Val Lys
   385 390 395 400
   His Glu Arg Ile His Thr Gly Val Lys Pro Tyr Gly Cys Thr Glu Cys
   405 410 415
   Gly Lys Ser Phe Ser His Gly His Gln Leu Thr Gln His Gln Lys Thr
   420 425 430
   His Ser Gly Ala Lys Ser Tyr Glu Cys Lys Glu Cys Gly Lys Ala Cys
   435 440 445
   Asn His Leu Asn His Leu Arg Glu His Gln Arg Ile His Asn Ser
   450 455 460